# EUROPEAN PATENT SPECIFICATION

(11) **EP 3 630 070 B1**
(45) Date of publication and mention of the grant of the patent: **02.12.2026**
(21) Application number: 18731684.9
(22) Date of filing: 25.05.2018
(51) Int. Cl.: A61K 9/16, A61K 9/20, A61K 31/202, A61K 31/397

(54) **FIXED DOSE FORMULATIONS**
FORMULIERUNGEN MIT FESTGELEGTER DOSIERUNG
FORMULES À DOSE FIXE

(30) Priority: 26.05.2017 US 201762511889 P; 29.12.2017 US 201715859279
(43) Date of publication of application: 08.04.2020
(73) Proprietor: Esperion Therapeutics, Inc., Ann Arbor, MI 48108 (US)
(72) Inventor: ABDELNASSER, Mohamed, Ann Arbor, MI 48108 (US); PILGAONKAR, Pratibha, S., Thane West 400604 (IN); GANDHI, Anilkumar S., Mumbai 400080 (IN)
(74) Representative: Mewburn Ellis LLP
(86) International application number: PCT/US2018/034646
(87) International publication number: WO 2018/218147

(56) References cited:
- WO-A1-2010/033179
- WO-A1-2017/023165
- US-A1- 2010 234 342
- "Encyclopedia of Pharmaceutical Technology", 1 January 2007, article ARMSTRONG N.A.: "Tablet manufacture", pages: 3653 - 3672, XP055449024

## Description

### BACKGROUND OF THE INVENTION

### Field of the invention

The present disclosure relates to formulations, kits, methods of use and methods for making pharmaceutical formulations comprising Bempedoic acid and Ezetimibe.

### Description of the Related Art

Certain therapeutic molecules belonging to categories or chemical classes have been identified, or rather acknowledged to possess poor flow and sticky bulk properties. Further, although not a formally expressed rule, chemists generally observe that compounds from Biopharmaceutical Classification System (BCS) class II are difficult to formulate owing to the fact that class II compounds are poorly water soluble and hence, suffer from poor dissolution in the gastrointestinal tract. Both Bempedoic acid (ETC-1002) and Ezetimibe are in the drug product BCS Class II compounds. Both are poorly soluble in water and highly permeable. In the solid state, Bempedoic acid exhibits poor flow characteristics and is very sticky. The observed stickiness adversely impacts various stages during development of pharmaceutical formulations including weighing, blending, granulation and compression. These problems adversely impact drug manufacturing operations, notably tablet compression (low rpm operation, weight variation, frequent machine stoppage; etc.). Standard granulation of Bempedoic acid only marginally reduces the sticky behavior thereby improving processability. Bempedoic acid also has a relatively low melting point, 88 - 91 °C, and as such contributes to the diminished plasticity of the bulk.

Formulation chemists have provided solutions; however, such work is unique to the particular drug being studied. A balance must be struck between stability and release characteristics such that adapted flow and other bulk physical properties meet pre-defined safety requirements for each API. This makes the art of API formulation very unpredictable. Thus, formulation chemists do not have a single universal set of rules or additives that enhance any given API's pharmacodynamic and/or bulk properties.
WO 2017/023165 A1 describes pharmaceutical compositions and therapeutic combinations comprising a cholesterol ester transfer protein (CETP) inhibitor described as 'Compound A', and an adenosine triphosphate citrate lyase (ACL) inhibitor/adenosine monophosphate-activated protein kinase (AMPK) activator which may be ETC-1002 (Bempedoic acid).

Accordingly, there is a need to develop stable and effective pharmaceutical compositions that allow for a formulation of Bempedoic acid and Ezetimibe that have improved, desirable PK and bulk physical properties.

The present disclosure overcomes difficulties associated with co-formulating Bempedoic acid and Ezetimibe, as described in detail below.

### SUMMARY OF THE INVENTION

The present invention is defined in the claims. The present invention provides a process of granulating Bempedoic acid, the process comprising: dry mixing: Bempedoic acid, a lubricant and a pharmaceutically acceptable excipient, wherein the lubricant is colloidal silicon dioxide; separately mixing an aqueous preparation of HPC-L; blending the dry mixture and aqueous preparation; and granulating the blend. The present invention also provides processes of manufacturing a monolayer tablet or a bilayer tablet comprising Ezetimibe and Bempedoic; the processes comprising said process of granulating Bempedoic acid as described in the claims.

Herein disclosed is the development of novel combination formulations for a drug product comprising Bempedoic acid (ETC-1002) and Ezetimibe. Also disclosed herein are granulated compositions of Bempedoic acid.

Two formulation options for the combination are identified as improved and compatible for both Bempedoic acid (ETC-1002) and Ezetimibe from a bioavailability study conducted and disclosed herein: a monolayer and a bilayer tablet formulation. The monolayer tablet is manufactured with granulated mixtures from both compounds blended together into a single layer. The bilayer tablet is manufactured with granulated mixtures from each compound compressed into two (2) separate layers.

The inventors have found that surface treatment of ETC-1002 with colloidal silicon dioxide reduces or eliminates the stickiness problem. This treatment involves blending ETC-1002 with colloidal silicon dioxide first, and then mixing the blend with hydroxypropyl cellulose (HPC-L) and microcrystalline cellulose in rapid mixer granulator; prior to granulation. Granulation is also carried with a binder solution comprising colloidal silicon dioxide and hydroxypropyl cellulose (HPC-L). The treatment of ETC-1002 and the preparation of the premix for granulation is carried out in such a way that: 1) excessive hydrophobicity is not imparted to the active, 2) the dissolution and release profile of ETC-1002 is not adversely impacted, 3) the stability of ETC-1002 is not adversely affected, and 4) incompatibility from any of the excipients does not arise in the fixed dose combination formulation containing Ezetimibe, particularly in the monolayer formulation.

### BRIEF DESCRIPTION OF THE DRAWINGS

FIG. 1 shows the Zetia dissolution profile for Ezetimibe in 500mL of dissolution medium using a USP Apparatus-II at 50 rpm.
FIG. 2 shows the dissolution profile of Bempedoic Acid tablet in different media with 2.0% w/v sodium lauryl sulfate (SLS).
FIG. 3 depicts the dissolution profile for Reference Product combinations at various dissolution conditions.
FIG. 4 is a graph of the dissolution profiles of the Reference Product and Fixed Dose Combination product in discriminatory dissolution media reflecting the difference in granulation processing.
FIG. 5 shows the dissolution profile of Bempedoic Acid in three different discriminatory media.
FIG. 6 is a graph showing the comparative dissolution profile of Bempedoic Acid Tablet (the Reference Product) vs. Fixed Dose Combination-monolayer tablets having Coarse and Fine Grade Bempedoic Acid.
FIG. 7 depicts a surface treatment of the granulated particles having Bempedoic Acid with Aerosil^{®} and HPC-L binder.
FIG. 8 is a graph showing the dissolution profile of tablets having different binder concentrations.
FIG. 9 depicts the comparative dissolution profile of a prototype Fixed Dose Combination tablet and a test Ezetimibe tablet (10mg) in discriminatory dissolution media.
FIG. 10 depicts the comparative dissolution profile for different batches of granulated Ezetimibe.
FIG. 11 illustrates the Fixed Dose Combination-monolayer tablet manufacturing process.
FIG. 12 illustrates the Fixed Dose Combination-bilayer tablet manufacturing process.
FIG. 13 shows comparative dissolution profiles for Ezetimibe from the monolayer and bilayer tablets against a Reference Product.
FIG. 14 shows comparative dissolution profiles of Bempedoic acid from the monolayer and bilayer tablets against a Reference Product.
FIG. 15 shows comparative dissolution profiles of Ezetimibe from the Fixed Dose Combination-monolayer tablet against an Ezetimibe test product.
FIG. 16 shows comparative dissolution profiles of Bempedoic acid from the monolayer and bilayer tablets against an Ezetimibe test product.
FIG. 17 shows comparative dissolution profiles of Bempedoic acid from the monolayer and bilayer tablets against an Ezetimibe test product.
FIG. 18 shows comparative dissolution profiles of Bempedoic acid from the Fixed Dose Combination test product in QC media.

### DETAILED DESCRIPTION OF THE INVENTION

Briefly, and as described in more detail below, described herein are novel compositions containing Bempedoic acid or Bempedoic acid and Ezetimibe, kits, methods of using and processes for making said compositions. The advantages for this approach are numerous and include, but are not limited to, improved pharmacokinetic (PK) properties of one or both of Bempedoic acid and Ezetimibe, and improved flowability and other bulk physiochemical properties of the composition in the solid state. As described above, many BCS class II compounds suffer from diminished PK and bulk properties. Hence, there is a significant need for formulations that improve the physiochemical properties of pharmaceutical compositions containing Bempedoic acid.

### Definitions

Terms used in the claims and specification are defined as set forth below unless otherwise specified. Further, if any term or symbol used herein is not defined as set forth below, it shall have its ordinary meaning in the art.

The practice of the present invention includes the use of conventional techniques of organic chemistry, molecular biology (including recombinant techniques), microbiology, cell biology, biochemistry and immunology, which are within the skill of the art.

As used herein and in the appended claims, singular articles such as "a," "an" and "the" and similar referents in the context of describing the elements (especially in the context of the following claims) are to be construed to cover both the singular and the plural, unless otherwise indicated herein or clearly contradicted by context. Recitation of ranges of values herein are merely intended to serve as a shorthand method of referring individually to each separate value falling within the range, including the upper and lower bounds of the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the embodiments and does not pose a limitation on the scope of the claims unless otherwise stated. No language in the specification should be construed as indicating any non-claimed element as essential.

As used herein, the term "cardiovascular diseases" refers to diseases of the heart and circulatory system. These diseases are often associated with dyslipoproteinemias and/or dyslipidemias. Cardiovascular diseases which the compositions of the present invention are useful for preventing or treating include but are not limited to arteriosclerosis; atherosclerosis; stroke; ischemia; endothelium dysfunctions, in particular those dysfunctions affecting blood vessel elasticity; peripheral vascular disease; coronary heart disease; myocardial infarction; cerebral infarction and restenosis.

As used herein, the term "dyslipidemias" refers to disorders that lead to or are manifested by aberrant levels of circulating lipids. To the extent that levels of lipids in the blood are too high, the compositions of the invention are administered to a patient to restore normal levels. Normal levels of lipids are reported in medical treatises known to those of skill in the art. For example, recommended blood levels of LDL, HDL, free triglycerides and other parameters relating to lipid metabolism can be found at the web site of the American Heart Association and that of the National Cholesterol Education Program of the National Heart, Lung and Blood Institute (http://www.americanheart.org/cholesterol- /about_level.html and http://www.nhlbi.nih.gov/health/public/heart/chol/hb-c_what.html, respectively). At the present time, the recommended level of HDL cholesterol in the blood is above 35 mg/dL; the recommended level of LDL cholesterol in the blood is below 130 mg/dL; the recommended LDL:HDL cholesterol ratio in the blood is below 5:1, ideally 3.5: 1; and the recommended level of free triglycerides in the blood is less than 200 mg/dL.

The term "subject" refers to any mammal including humans, and so includes mammals such as those animals of veterinary and research interest that are including, but not limited to: simians, cattle, horses, dogs, cats, and rodents. The term "subject" is interchangeable with the term "patient".

The term "sufficient amount" means an amount sufficient to produce a desired effect, e.g., an amount sufficient to modulate protein aggregation in a cell.

The term "therapeutically effective amount" is an amount that is effective to ameliorate a symptom of a disease. A therapeutically effective amount can, in some embodiments, be a "prophylactically effective amount" as prophylaxis can be considered therapy.

The term "administering" or "administration" of' a drug and/or therapy to a subject (and grammatical equivalents of this phrase) refers to both direct or indirect administration, which may be administration to a subject by a medical professional, may be self-administration, and/or indirect administration, which may be the act of prescribing or inducing one to prescribe a drug and/or therapy to a subject.

The term "treating" or "treatment of" a disorder or disease refers to taking steps to alleviate the symptoms of the disorder or disease, or otherwise obtain some beneficial or desired results for a subject, including clinical results. Any beneficial or desired clinical results may include, but are not limited to, alleviation or amelioration of one or more symptoms of cancer or conditional survival and reduction of tumor load or tumor volume; diminishment of the extent of the disease; delay or slowing of the tumor progression or disease progression; amelioration, palliation, or stabilization of the tumor and/or the disease state; or other beneficial results.

The compounds of the present technology can exist as solvates, especially hydrates. Hydrates may form during manufacture of the compounds or compositions comprising the compounds, or hydrates may form over time due to the hygroscopic nature of the compounds. Compounds of the present technology can exist as organic solvates as well, including DMF, ether, and alcohol solvates among others. The identification and preparation of any particular solvate is within the skill of the ordinary artisan of synthetic organic or medicinal chemistry.

For the purposes of this specification and appended claims, unless otherwise indicated, all numbers expressing amounts, sizes, dimensions, proportions, shapes, formulations, parameters, percentages, parameters, quantities, characteristics, and other numerical values used in the specification and claims, are to be understood as being modified in all instances by the term "about" even though the term "about" may not expressly appear with the value, amount or range. Accordingly, unless indicated to the contrary, the numerical parameters set forth in the following specification and attached claims are not and need not be exact, but may be approximate and/or larger or smaller as desired, reflecting tolerances, conversion factors, rounding off, measurement error and the like, and other factors known to those of skill in the art depending on the desired properties sought to be obtained by the presently disclosed subject matter. For example, the term "about," when referring to a value can be meant to encompass variations of ± 100%, such as ± 50%, such as ± 20%, such as ± 10%, such as ± 5%, such as ±1%, such as ± 0.5%, such as ± 0.1% from the specified amount, as such variations are appropriate to perform the disclosed methods or employ the disclosed compositions.

Unless defined otherwise, all technical and scientific terms used herein have the meaning commonly understood by a person skilled in the art to which this invention belongs.

### Formulations and Pharmaceutical compositions

Herein disclosed are combinations of Bempedoic acid and Ezetimibe formulated in pharmaceutical compositions.

The present disclosure provides for a pharmaceutical composition comprising: Bempedoic acid admixed with a lubricant wherein the lubricant is colloidal silicon dioxide; Ezetimibe; and a pharmaceutically acceptable excipient.

The present disclosure provides for a pharmaceutical composition wherein the composition comprises at least 40% and nor more than 95% Bempedoic acid by weight of the total composition and at least 0.5% and no more than 20% Ezetimibe by weight of the total composition.

The present disclosure provides for a pharmaceutical composition wherein the composition further comprises one or more of: magnesium stearate, hydroxypropyl cellulose (HPC-L), a pyrrolidone compound, a saccharide, an anionic surfactant, microcrystalline cellulose and a starch.

The present disclosure provides for a pharmaceutical composition wherein the composition further comprises each one of: magnesium stearate, hydroxypropyl cellulose (HPC-L), a pyrrolidone compound, a saccharide, an anionic surfactant, microcrystalline cellulose and a starch.

The present disclosure provides for a pharmaceutical composition wherein the microcrystalline cellulose, when present, comprises an average particle size of at least 100 µm and comprises a moisture content at least 3% and no more than 5% by weight of the microcrystalline cellulose.

The present disclosure provides for a pharmaceutical composition wherein the anionic surfactant, when present, is sodium lauryl sulfate.

The present disclosure provides for a pharmaceutical composition wherein the pyrrolidone compound, when present, is Povidone.

The present disclosure provides for a pharmaceutical composition wherein the saccharide, when present, is lactose monohydrate.

The present disclosure provides for a pharmaceutical composition wherein 1.03% by weight of the total magnesium stearate when present, has a particle size at least 45 µm and no more than 150 µm.

The present disclosure provides for a pharmaceutical composition wherein the composition is in the form of a tablet and further comprises a polyvinyl alcohol (PVA) based coating; and wherein the coating comprises: polyvinyl alcohol (PVA), glycerol monocaprylocaprate type 1, sodium lauryl sulfate, titanium dioxide and talc.

The present disclosure provides for a pharmaceutical composition wherein the amount of magnesium stearate is between 1 mg and 10 mg, the amount of hydroxypropyl cellulose (HPC-L) is between 5 mg and 25 mg, the amount of pyrrolidone compound is between 0.5 mg and 5 mg, the amount of saccharide is between 50 mg and 100 mg, the amount of anionic surfactant is between 0.5 mg and 5 mg, the amount of microcrystalline cellulose is between 25 mg and 100 mg and the amount of sodium starch glycolate is between 5 mg and 50 mg.

The present disclosure provides for a pharmaceutical composition wherein the amount of Bempedoic acid is between 80 mg and 250 mg; and the amount of Ezetimibe is between 5 mg and 25 mg. The amount of Bempedoic acid may be between 100 mg and 200 mg; and the amount of Ezetimibe is between 7 mg and 15 mg. The amount of Bempedoic acid may be between 150 mg and 200 mg; and the amount of Ezetimibe is between 9 mg and 12 mg.

The present disclosure provides for a pharmaceutical composition wherein the amount of Bempedoic acid is 180 mg and the amount of Ezetimibe is 10 mg.

The present disclosure provides for a pharmaceutical composition wherein the amount of Bempedoic acid is a fixed dose and the amount of Ezetimibe is a fixed dose.

The present disclosure provides for a pharmaceutical composition comprising Ezetimibe and Bempedoic acid as described herein that has improved flowability characteristics as described herein.

The present disclosure provides for a pharmaceutical composition comprising Ezetimibe and Bempedoic acid as described herein that has improved non-stickiness characteristics as described herein.

The present disclosure provides for a pharmaceutical composition comprising Ezetimibe and Bempedoic acid as described herein that has improved PK characteristics as described herein.

The present disclosure provides for a pharmaceutical composition comprising Ezetimibe and Bempedoic acid as described herein that has improved solubility characteristics as described herein.

The present disclosure provides for a pharmaceutical composition comprising Ezetimibe and Bempedoic acid as described herein that has improved extended release characteristics as described herein.

The present disclosure provides for a pharmaceutical composition comprising Ezetimibe and Bempedoic acid as described herein that has improved chemo-physical characteristics such as particle size, surface area, pore volume and other properties as described herein.

Ezetimibe in the pharmaceutical composition may be amorphous. Ezetimibe in the pharmaceutical composition may be a polymorph.

Bempedoic acid in the pharmaceutical composition may be amorphous. Bempedoic acid in the pharmaceutical composition may be a polymorph.

One of Bempedoic acid or Ezetimibe may be amorphous. One of Bempedoic acid or Ezetimibe may be a polymorph.

Formulations disclosed herein comprise the active compound(s), a pharmaceutically acceptable excipient, carrier, buffer, stabiliser or other materials well known to those skilled in the art. Such materials should be non-toxic and should not interfere with the efficacy of the active ingredient. The precise nature of the carrier or other material can depend on the route of administration, e.g. oral, intravenous, cutaneous or subcutaneous, nasal, intramuscular, intraperitoneal routes.

The compounds in the combination, and solvates thereof, of the present disclosure can be formulated in pharmaceutical compositions. These compositions can comprise a pharmaceutically acceptable excipient, carrier, buffer, stabilizer or other materials well known to those skilled in the art. The precise nature of the carrier or other material can depend on the route of administration, e.g. oral, intravenous, cutaneous or subcutaneous, nasal, intramuscular, intraperitoneal routes.

Pharmaceutical compositions for oral administration can be in tablet, capsule, powder or liquid form. A tablet can include a solid carrier such as gelatin or an adjuvant. Liquid pharmaceutical compositions generally include a liquid carrier such as water, petroleum, animal or vegetable oils, mineral oil or synthetic oil. Physiological saline solution, dextrose or other saccharide solution or glycols such as ethylene glycol, propylene glycol or polyethylene glycol can be included.

For intravenous, cutaneous or subcutaneous injection, or injection at the site of affliction, the active ingredient will be in the form of a parenterally acceptable aqueous solution which is pyrogen-free and has suitable pH, isotonicity and stability. Those of relevant skill in the art are well able to prepare suitable solutions using, for example, isotonic vehicles such as Sodium Chloride Injection, Ringer's Injection, Lactated Ringer's Injection. Preservatives, stabilisers, buffers, antioxidants and/or other additives can be included, as required.

A composition can be administered alone or in combination with other treatments, either simultaneously or sequentially dependent upon the condition to be treated.

In general, the compounds of the present technology will be administered in a therapeutically effective amount by any of the accepted modes of administration for agents that serve similar utilities. The actual amount of the compound of the present technology, i.e., the active ingredient, will depend upon numerous factors such as the severity of the disease to be treated, the age and relative health of the subject, the potency of the compound used, the route and form of administration, and other factors well known to the skilled artisan. The drug can be administered at least once a day, preferably once or twice a day.

An effective amount of such agents can readily be determined by routine experimentation, as can the most effective and convenient route of administration and the most appropriate formulation. Various formulations and drug delivery systems are available in the art. See, e.g., Gennaro, A.R., ed. (1995) Remington's Pharmaceutical Sciences, 18th ed., Mack Publishing Co.

A therapeutically effective dose can be estimated initially using a variety of techniques well-known in the art. Initial doses used in animal studies may be based on effective concentrations established in cell culture assays. Dosage ranges appropriate for human subjects can be determined, for example, using data obtained from animal studies and cell culture assays.

An effective amount or a therapeutically effective amount or dose of an agent, *e.g.,* a compound of the present technology, refers to that amount of the agent or compound that results in amelioration of symptoms or a prolongation of survival in a subject. Toxicity and therapeutic efficacy of such molecules can be determined by standard pharmaceutical procedures in cell cultures or experimental animals, e.g., by determining the LD₅₀ (the dose lethal to 50 % of the population) and the ED₅₀ (the dose therapeutically effective in 50 % of the population). The dose ratio of toxic to therapeutic effects is therapeutic index, which can be expressed as the ratio LD₅₀/ ED₅₀. Agents that exhibit high therapeutic indices are preferred.

The effective amount or therapeutically effective amount is the amount of the compound or pharmaceutical composition that will elicit the biological or medical response of a tissue, system, animal or human that is being sought by the researcher, veterinarian, medical doctor or other clinician. Dosages particularly fall within a range of circulating concentrations that includes the ED₅₀ with little or no toxicity. Dosages may vary within this range depending upon the dosage form employed and/or the route of administration utilized. The exact formulation, route of administration, dosage, and dosage interval should be chosen according to methods known in the art, in view of the specifics of a subject's condition.

Dosage amount and interval may be adjusted individually to provide plasma levels of the active moiety that are sufficient to achieve the desired effects; i.e., the minimal effective concentration (MEC). The MEC will vary for each compound but can be estimated from, for example, in vitro data and animal experiments. Dosages necessary to achieve the MEC will depend on individual characteristics and route of administration. In cases of local administration or selective uptake, the effective local concentration of the drug may not be related to plasma concentration.

The amount of agent or composition administered may be dependent on a variety of factors, including the sex, age, and weight of the subject being treated, the severity of the affliction, the manner of administration, and the judgment of the prescribing physician.

The present technology is not limited to any particular composition or pharmaceutical carrier, as such may vary. In general, compounds of the present technology will be administered as pharmaceutical compositions by any one of the following routes: oral, systemic (e.g., transdermal, intranasal or by suppository), or parenteral (e.g., intramuscular, intravenous or subcutaneous) administration. The preferred manner of administration is oral using a convenient daily dosage regimen that can be adjusted according to the degree of affliction. Compositions can take the form of tablets, pills, capsules, semisolids, powders, sustained release formulations, solutions, suspensions, elixirs, aerosols, or any other appropriate compositions. Another preferred manner for administering compounds of the present technology is inhalation.

The choice of formulation depends on various factors such as the mode of drug administration and bioavailability of the drug substance. For delivery via inhalation the compound can be formulated as liquid solution, suspensions, aerosol propellants or dry powder and loaded into a suitable dispenser for administration. There are several types of pharmaceutical inhalation devices-nebulizer inhalers, metered dose inhalers (MDI) and dry powder inhalers (DPI). Nebulizer devices produce a stream of high velocity air that causes therapeutic agents (which are formulated in a liquid form) to spray as a mist that is carried into the subject's respiratory tract. MDI's typically are formulation packaged with a compressed gas. Upon actuation, the device discharges a measured amount of therapeutic agent by compressed gas, thus affording a reliable method of administering a set amount of agent. DPI dispenses therapeutic agents in the form of a free flowing powder that can be dispersed in the subject's inspiratory air-stream during breathing by the device. In order to achieve a free flowing powder, therapeutic agent is formulated with an excipient such as lactose. A measured amount of therapeutic agent is stored in a capsule form and is dispensed with each actuation.

Pharmaceutical dosage forms of a compound of the present technology may be manufactured by any of the methods well-known in the art, such as, for example, by conventional mixing, sieving, dissolving, melting, granulating, dragee-making, tabletting, suspending, extruding, spray-drying, levigating, emulsifying, (nano/micro-) encapsulating, entrapping, or lyophilization processes. As noted above, the compositions of the present technology can include one or more physiologically acceptable inactive ingredients that facilitate processing of active molecules into preparations for pharmaceutical use.

### Tablets

The present disclosure provides for a monolayer or a bilayer tablet as described herein. The monolayer or a bilayer tablet comprise a composition of Bempedoic acid and Ezetimibe, and optionally one or more pharmaceutically acceptable excipients as described herein.

The present disclosure provides for a bilayer tablet comprising Bempedoic acid and Ezetimibe, wherein the first layer comprises: Ezetimibe granulated with a pharmaceutically acceptable excipient; and wherein the second layer comprises: Bempedoic acid granulated with a lubricant and a pharmaceutically acceptable excipient, wherein the lubricant is colloidal silicon dioxide.

The present disclosure provides for a bilayer tablet wherein the Bempedoic acid is at least 20% and no more than 64% by weight of the total tablet and Ezetimibe is at least 1% and no more than 7% by weight of the total tablet.

The present disclosure provides for a bilayer tablet wherein the first layer is at least 0.1% and no more than 23% by weight of the total tablet and the second layer is at least 0.1% and no more than 74% by weight of the total tablet.

The present disclosure provides for a bilayer tablet wherein the Friability of the tablet is at least 0.01% and no more than 0.1%. Friability is a routine test for tablet compositions, the skilled artisan can determine Friability by a number of methods. For example, the skilled artisan may determine Friability of compositions of the present disclosure by the methods described in the monograph USP Tablet Friability < 1216>, which describes the recommended apparatus and the test procedure.

The present disclosure provides for a monolayer or a bilayer tablet comprising Ezetimibe and Bempedoic acid as described herein that has improved physical characteristics such as Friability and other properties as described herein.

### Granulated compositions

The present disclosure provides for a granulated composition comprising: Bempedoic acid admixed with a lubricant wherein the lubricant is colloidal silicon dioxide.

The present disclosure provides for a granulated composition wherein the composition further comprises a pharmaceutically acceptable excipient.

The present disclosure provides for a granulated composition wherein the composition has a bulk density of at least 0.25 gm/ml and no more than 0.55 gm/ml.

The present disclosure provides for a granulated composition wherein the composition has a Carr's Index of at least 10 and no more than 30. The Carr index relates to the compressibility and hence, the flowability of a material. Carr's index is a routine measurement for one skilled in the art and numerous methods may be employed. For example, the skilled artisan can use the methods, apparatus and procedures described in the monograph USP29-NF24 (page 2638) to determine the Carr's Index of a composition of the present disclosure.

The present disclosure provides for a granulated composition wherein the granules of the composition have an angle of repose of at least 20 no more than 45. The morphology of a given material and its composition both affect the angle of repose. The angle of repose is related to the density, surface area, shapes of the particles, and the coefficient of friction of the material. One skilled in the art can use numerous methods to determine the angle of repose, one example would be to use the methods and procedures described in USP29.

The present disclosure provides for a granulated composition wherein the Bempedoic acid is present in an amount of at least 50% and no more than 95% by weight of the total formulation.

The present disclosure provides for a granulated composition wherein the composition further comprises hydroxypropyl cellulose (HPC-L). The present disclosure provides for a granulated composition wherein the composition further comprises microcrystalline cellulose. The amount of the HPC-L may be at least 3% and no more than 10% by weight of the total formulation; the amount of the Bempedoic acid may be at least 50% and no more than 95% by weight of the total formulation; and the amount of the microcrystalline cellulose may be at least 1% and no more than 20% by weight of the total formulation.

Bempedoic acid in the granulated composition may be amorphous. Bempedoic acid in the granulated composition may be a polymorph.

### Kits

The present disclosure also provides for a kit comprising one or more compositions which itself comprises Bempedoic acid or a combination of Bempedoic acid and Ezetimibe, and instructions for use. The present disclosure further provides for a kit comprising one or more compositions.

The present disclosure further provides for a kit comprising one or more compositions comprising Bempedoic acid or Bempedoic acid and Ezetimibe, and optionally the composition and/or kit includes at least one pharmaceutically acceptable carrier or excipient.

The disclosure provides for a kit comprising a combination composition comprising: Bempedoic acid admixed with a lubricant wherein the lubricant is colloidal silicon dioxide, and Ezetimibe, and optionally at least one pharmaceutically acceptable carrier or excipient.

The present disclosure provides for a kit and instructions for use, where the instructions for use recite a process or recite directions for mixing the one or more of the granulated compositions or one or more pharmaceutical compositions or one or more tablets with one or more compositions.

Individual components of the kit can be packaged in separate containers and, associated with such containers, can be a notice in the form prescribed by a governmental agency regulating the manufacture, use or sale of pharmaceuticals or biological products, which notice reflects approval by the agency of manufacture, use or sale. The kit may optionally contain instructions or directions outlining the method of use or administration regimen for the antigen-binding construct.

When one or more components of the kit are provided as solutions, for example an aqueous solution, or a sterile aqueous solution, the container means may itself be an inhalant, syringe, pipette, eye dropper, or other such like apparatus, from which the solution may be administered to a subject or applied to and mixed with the other components of the kit.

The components of the kit may also be provided in dried or lyophilized form and the kit can additionally contain a suitable solvent for reconstitution of the lyophilized components. Irrespective of the number or type of containers, the kits described herein also may comprise an instrument for assisting with the administration of the composition to a patient. Such an instrument may be an inhalant, nasal spray device, syringe, pipette, forceps, measured spoon, eye dropper or similar medically approved delivery vehicle.

### Syntheses of Bempedoic acid (ETC-1002) and Ezetimibe

The structure of ETC-1002 is:

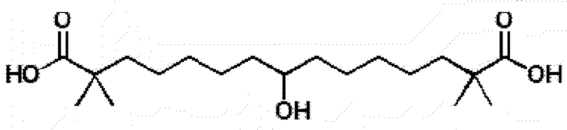

ETC-1002 and the processes for the synthesis of ETC-1002 are disclosed in issued U.S. Patent No. 7,335,799. The details of this process can be found in the published U.S. Patent Publication No. US2005/0043278A1, in paragraphs [0247] - [0343] of the specification.

The structure of Ezetimibe is:

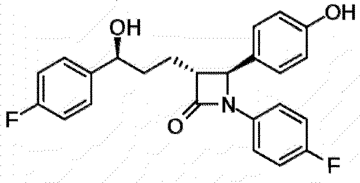

Ezetimibe and the process for the synthesis of Ezetimibe is disclosed in the issued U.S. Patent No. 5,631,365. The details of this process can be found in the specification, beginning on page 4 right column, line 43 through page 11 right column, line 65.

Additionally, both compounds are small molecules under 500 Da in weight and the skilled artisan will be able to use synthetic reference texts to synthesize the desired final compound from readily available or commercially available chemicals. Such references include, but are not limited to: as Fieser and Fieser's Reagents for Organic Synthesis, Volumes 1-15 (John Wiley, and Sons, 1991), Rodd's Chemistry of Carbon Compounds, Volumes 1-5, and Supplementals (Elsevier Science Publishers, 1989), Organic Reactions, Volumes 1-40 (John Wiley, and Sons, 1991), March's Advanced Organic Chemistry, (John Wiley, and Sons, 5th Edition, 2001), and Larock's Comprehensive Organic Transformations (VCH Publishers Inc., 1989), T. W. Greene and P.G.M. Wuts, Protecting Groups in Organic Synthesis, Third Edition, Wiley, New York, 1999.

### Granulating

In some aspects, the present disclosure provides for a process of granulating Bempedoic acid, the process comprising: dry mixing: Bempedoic acid, a lubricant and a pharmaceutically acceptable excipient, wherein the lubricant is colloidal silicon dioxide; separately mixing an aqueous preparation of HPC-L; blending the dry mixture and aqueous preparation; and granulating the blend.

In some aspects, the present disclosure provides for a process of granulating Bempedoic acid, wherein the process further comprises using a rapid mixer to granulate the blend.

In some aspects, the present disclosure provides for a process of granulating Bempedoic acid, wherein the process further comprises drying the blend.

In some aspects, the present disclosure provides for a process of granulating Bempedoic acid, wherein the process further comprises milling and co-sifting the blend.

In some aspects, the present disclosure provides for a process of granulating Bempedoic acid, wherein the dry mixing is carried out for at least forty-five (45) minutes.

In some aspects, the present disclosure provides for a process of granulating Bempedoic acid, wherein the dry mixing is carried out for no more than twenty four (24) hours in duration.

### Manufacturing

In some aspects, the present disclosure provides for a process of manufacturing a monolayer tablet comprising Ezetimibe and Bempedoic acid, the process comprising: (a) a process of granulating Bempedoic acid, the process comprising: dry mixing: Bempedoic acid, a lubricant and a pharmaceutically acceptable excipient, wherein the lubricant is colloidal silicon dioxide; separately mixing an aqueous preparation of HPC-L; blending the dry mixture and aqueous preparation; and granulating the blend to obtain the Bempedoic acid granules; (b) granulating a composition comprising Ezetimibe to obtain Ezetimibe granules; (c) blending the Ezetimibe and Bempedoic acid granules together to obtain a granulated mixture; (d) compressing the granulated mixture into a single layer; and (e) coating the single layer to obtain the monolayer tablet.

In some aspects, the present disclosure provides for a process of manufacturing a monolayer tablet wherein the process further comprises drying the tablets.

In some aspects, the present disclosure provides for a process of manufacturing a monolayer tablet wherein the coating comprises one or more of: PVA, glycerol monocaprylocaprate type 1, sodium lauryl sulfate, titanium dioxide and talc.

In some aspects, the present disclosure provides for a process of manufacturing a monolayer tablet wherein the coating comprises each one of: PVA, glycerol monocaprylocaprate type 1, sodium lauryl sulfate, titanium dioxide and talc.

In some aspects, the present disclosure provides for a process of manufacturing a bilayer tablet comprising Ezetimibe and Bempedoic acid, the process comprising: (a) a process of granulating Bempedoic acid, the process comprising: dry mixing: Bempedoic acid, a lubricant and a pharmaceutically acceptable excipient, wherein the lubricant is colloidal silicon dioxide; separately mixing an aqueous preparation of HPC-L; blending the dry mixture and aqueous preparation; and granulating the blend to obtain the Bempedoic acid granules; (b) granulating a composition comprising Ezetimibe to obtain Ezetimibe granules; (c) blending the Ezetimibe granules with a pharmaceutically acceptable excipient; (d) blending the Bempedoic acid granules with a pharmaceutically acceptable excipient; (e) compressing the Ezetimibe and Bempedoic acid blends into a single composition containing two (2) separate layers; and (f) coating the composition.

In some aspects, the present disclosure provides for a process of manufacturing a bilayer tablet wherein the process further comprises drying the tablets.

In some aspects, the present disclosure provides for a process of manufacturing a bilayer tablet wherein the Ezetimibe composition includes an anionic surfactant.

### Methods of use

The references to the methods of treatment of this description are to be interpreted as references to compounds, pharmaceutical compositions and medicaments for use in said methods of treatment.

The present disclosure provides for methods for the treatment or prevention of a cardiovascular disease, said methods comprising administering a pharmaceutical composition comprising: Ezetimibe and Bempedoic acid admixed with a lubricant wherein the lubricant is colloidal silicon dioxide.

The present disclosure provides for methods for the treatment or prevention of a cardiovascular disease, said methods comprising administering a pharmaceutical composition comprising: Bempedoic acid admixed with a lubricant wherein the lubricant is colloidal silicon dioxide; Ezetimibe; and a pharmaceutically acceptable excipient to a subject in need thereof.

The present disclosure provides for methods for the treatment or prevention of a cardiovascular disease, said methods comprising administering a pharmaceutical composition comprising: a fixed dose of Bempedoic acid admixed with a lubricant wherein the lubricant is colloidal silicon dioxide; a fixed dose of Ezetimibe; and a pharmaceutically acceptable excipient to a subject in need thereof.

The present disclosure provides for methods for the treatment or prevention of a cardiovascular disease, said methods comprising administering a fixed dose of a pharmaceutical composition disclosed herein.

The present disclosure provides for methods for the treatment or prevention of a dyslipidemia, said methods comprising administering a pharmaceutical composition comprising: Ezetimibe and Bempedoic acid admixed with a lubricant wherein the lubricant is colloidal silicon dioxide.

The present disclosure provides for methods for the treatment or prevention of a dyslipidemia, said methods comprising administering a pharmaceutical composition comprising: Bempedoic acid admixed with a lubricant wherein the lubricant is colloidal silicon dioxide; Ezetimibe; and a pharmaceutically acceptable excipient to a subject in need thereof.

Dyslipidemias which the compositions of the present invention are useful for preventing or treating include but are not limited to hyperlipidemia and low blood levels of high density lipoprotein (HDL) cholesterol. In certain embodiments, the hyperlipidemia for prevention or treatment by the compounds of the present invention is familial hypercholesterolemia; familial combined hyperlipidemia; reduced or deficient lipoprotein lipase levels or activity, including reductions or deficiencies resulting from lipoprotein lipase mutations; hypertriglyceridemia; hypercholesterolemia; high blood levels of urea bodies (e.g. .beta.-OH butyric acid); high blood levels of Lp(a) cholesterol; high blood levels of low density lipoprotein (LDL) cholesterol; high blood levels of very low density lipoprotein (VLDL) cholesterol and high blood levels of non-esterified fatty acids.

The present disclosure provides for methods for altering lipid metabolism in a patient, e.g., reducing LDL in the blood of a patient, reducing free triglycerides in the blood of a patient, increasing the level of high density lipoprotein (HDL) in the blood of a patient, reducing the level of very low density lipoprotein (VLDL) in the blood of a patient, reducing the number of very low density lipoprotein (VLDL) particles in the blood of a patient, reducing the size of VLDL particles in the blood of a patient, increasing the level of apolipoprotein A-1 (ApoA1) in the blood of a patient, reducing the he ratio of apolipoprotein B (ApoB) to apolipoprotein A-1 (ApoA1) in the blood of a patient, increasing the ratio of HDL to LDL in the blood of a patient, and inhibiting saponified and/or non-saponified fatty acid synthesis, said methods comprising administering to the patient a pharmaceutical composition comprising: Bempedoic acid admixed with a lubricant wherein the lubricant is colloidal silicon dioxide; Ezetimibe; and a pharmaceutically acceptable excipient in an amount effective to alter lipid metabolism.

### EXAMPLES

Formulation development was initiated with the characterization of both APIs along with an evaluation of individual reference products. Dissolution profile, and drug-drug and drug-excipient compatibility were characterized. Additionally, the process operations were defined and a process control strategy was examined. Manufacturing process developments identified wet granulation, blending, compression, and coating as the main process options. Risk was assessed throughout development to identify high risk formulations and process variables and to determine a path forward to develop a control strategy. Risk assessments were updated after development to capture the reduction of risk due to improved product and process understanding.

Dissolution parameters (e.g. dissolution media, volume, apparatus, and agitation speed) were selected based on the pending USP monograph for Ezetimibe and the analytical methods listed in IND # 106,654 for Bempedoic acid. The particle size of Ezetimibe is critical for dissolution; hence the micronized form of each API was used. Limits of single known and unspecified impurities were set in accordance with ICH Q3B (R2) to control impurity in finished drug products.

The quality target product profile (QTPP) is defined based on properties of the drug substance, characterization of the reference product, and other information available for the two compounds. Critical quality attributes (CQAs) were identified as those drug product attributes that could impact the risk to a patient (safety and efficacy). The present disclosure focuses on those CQAs that are impacted to affect a realistic change to the drug product formulation or manufacturing process, namely: assay, content uniformity, dissolution, and degradation products.

Initially, it was found that Bempedoic acid exhibits sticky behavior as a granulated material. This behavior leads to extraordinary challenges to making tablets by compression. To address this challenge, the granulation process was modified by treating the active with a mixture of colloidal silicon and a cellulosic binder.

Granulation of bempedoic acid is altered with a mixture of colloidal silicon dioxide and hydroxypropyl cellulose (HPC-L). This blend was further granulated using a solution of HPC-L. The solubility of Ezetimibe was improved by homogenization with sodium lauryl sulfate (SLS) and Povidone. This dispersion was then used to granulate excipient blend using top spray attachment of fluid bed processor. Opadry AMBII white 88A180040 was selected as the coating agent to avoid the incompatibility of ezetimibe with polyethylene glycol and/or polyvinyl alcohol.

For the purposes of this development, drug products containing bempedoic acid film coated tablet (180 mg) from Esperion Therapeutics, Inc. and Zetia^{®} (Ezetimibe, 10 mg) manufactured by MSD International GmbH (NDA No: 21445) were utilized as the reference products.

The objective was broken down into the following activities: Study the compatibility of both drug substances together and with selected excipients; Develop a product with a satisfactory process and stability profile; Match the release profile of FDC product to individual reference product in QC and discriminatory dissolution media. Study the pharmacokinetics of the developed FDC product along with the reference product (co-administered ETC- 1002 (180 mg) tablets and Zetia (10 mg).

Zetia^{®} (ezetimibe): Zetia is commercially available as a tablet for oral administration containing 10 mg of Ezetimibe. It was approved in the United States in 2002 (NDA No: 21445).

Bempedoic acid (ETC-1002) tablet: Bempedoic acid (ETC-1002) is an innovative, first-in-class, small molecule designed to lower elevated levels of LDL-C and to avoid side effects associated with existing LDL-C lowering therapies. It is intended to be taken once a day.

### Example 1

### Drug Release

Both ezetimibe and Bempedoic acid are BCS Class-II compounds (poorly soluble and highly permeable) and therefore drug release is a rate limiting process for absorption. A thorough evaluation of the drug release profile of the reference products was carried out.

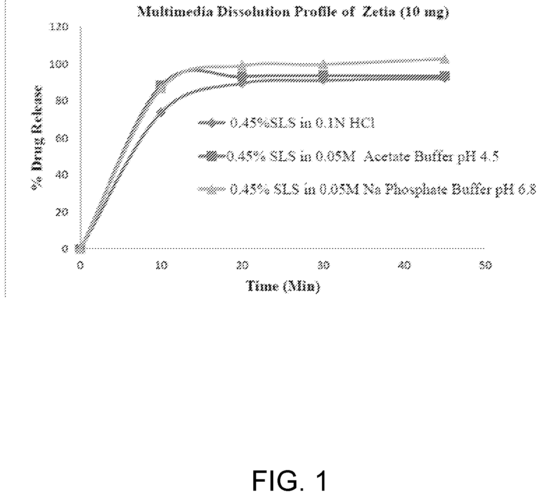

Multimedia Dissolution of Zetia: Dissolution characterization was performed in accordance with pending USP monograph (500 mL 0.45% SLS in 0.05 M acetate buffer, pH 4.5 dissolution media, Apparatus-II, 50 rpm). The temperature of the dissolution media was maintained at 37 ± 0.5°C and the released drug concentration was determined using HPLC. The drug release was also studied in two additional media at alternate pH (0.1 N HCl with 0.45% w/v SLS and pH 6.8 phosphate buffer with 0.45% w/v SLS).. Multimedia dissolution profiles are shown in Table 1 and Figure 1.

**Table 1: Multimedia Dissolution Data for Zetia**

| **Product** | **Zetia** | | |
|---|---|---|---|
| **Media** | **0.45% SLS in 0.1N HCl** | **0.45% SLS in 0.05M Acetate Buffer pH 4.5** | **0.45% SLS in 0.05M Na Phosphate Buffer pH 6.8** |
| **Time (min)** | **% Drug release** | | |
| 10 | 73.6 | 88.4 | 86.5 |
| 20 | 89.5 | 93.2 | 99.1 |
| 30 | 91.2 | 93.7 | 99.8 |
| 45 | 92.3 | 93.5 | 102.7 |

Zetia exhibited rapid dissolution with more than 80% drug release within 15 minutes in all three studied media. The aqueous solubility of ezetimibe is very low; however the use of 0.45% SLS enables sink condition.

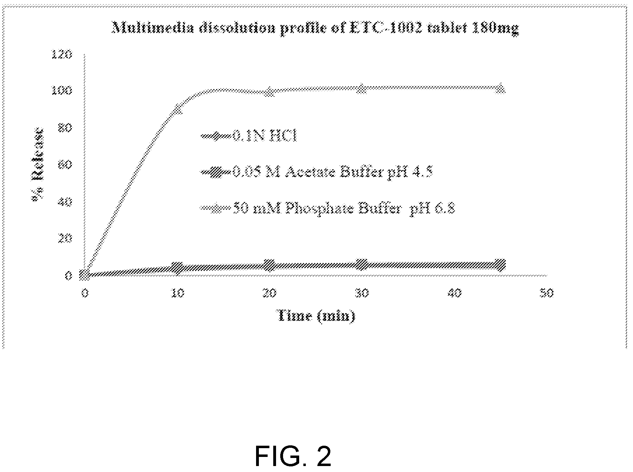

Multimedia Dissolution of Bempedoic Acid Tablets. Dissolution characterization was performed in accordance with the dissolution method detailed in IND # 106,654. The dissolution conditions were 900 mL phosphate buffer, pH 6.8, using Apparatus-II at 50 rpm. The data are presented in Table 2 and Figure 2.

**Table 2: Multimedia dissolution data for Bempedoic Acid tablets**

| **Product** | **Bempedoic Acid Tablets** | | |
|---|---|---|---|
| **Media** | **0.1N HCl** | **Acetate Buffer pH 4.5** | **Phosphate Buffer pH 6.8** |
| **Time (min)** | **% Drug release** | | |
| 10 | 2.9 | 4.2 | 90.2 |
| 20 | 4.4 | 5.4 | 99.6 |
| 30 | 5.1 | 5.9 | 101.6 |
| 45 | 4.4 | 6.2 | 101.8 |

Bempedoic acid has two carboxylic acidic functional groups (-COOH) which make solubility pH dependent, as observed in this multimedia dissolution study. Low dissolution values were observed in 0.1 N HCl and acetate buffer, pH 4.5, however in the QC medium (phosphate buffer, pH 6.8) more than 85% of the drug was released in 20 minutes.

The physicochemical characterizations of the reference products are summarized in Table 3.

**Table 3: Physical Attributes of Reference Products**

| **Parameter** | | **Zetia** | **Bempedoic Acid Tablets** |
|---|---|---|---|
| **Brand Name** | | Zetia^{®}(ezetimibe) | Not Applicable |
| **Generic Name** | | Ezetimibe 10 mg | Bempedoic acid 180 mg |
| **Manufacturer** | | MSD International GmbH, Inc., a subsidiary of Merck & Co. Inc. | Esperion Therapeutic. Inc. |
| **Dosage form** | | Tablet | Tablet |
| **Label claim** | | Each uncoated tablet contain 10 mg ezetimibe | Each film coated tablet contain 180 mg bempedoic acid |
| **Batch No.** | | L015901 | N460335 |
| **Expiry date** | 12-2017 | | Not available |
| **Pack** | HDPE (NDC 66582-414-31) Pack of 30 tablets | | HDPE container Pack of 30 tablets |
| **Appearance** | White elongated beveled tablet debossed "414" on one side and plain on other side | | Oval, white to off white film coated tablet debossed with "ABC" on one face and debossed with 000 on the opposite face |
| **Coated/Uncoated** | Uncoated | | Coated |
| **Average weight (mg)** | 100.2 | | 310.3 |
| **Length (mm)** | 8.24 - 8.26 | | 14.03 - 14.06 |
| **Thickness (mm)** | 4.65 - 4.67 | | 4.62 - 4.63 |
| **Hardness (N)** | 48 - 50 | | 125 - 135 |
| **Disintegration Time** | 3 - 4 min | | 5 - 6 min |
| **pH (1 tablet in 100 mL of Purified water)** | 6.8 | | 6.3 |
| **Water Content bv Karl Fischer (%w/v)** | 2.46 | | 5.48 |
| **Assay (%)** | 100.0 | | 99.8 |
| **Related Substance** | Impurity | % | No Unspecified impurities detected |
| | Cyclic ether | 0.07 | |
| | RRS isomer | Not detected | |
| | EZT⁽¹⁾ 21 (Keto impurity) | BLOQ | |
| | Single maximum unspecified impurities | 0.06 | |
| | Total impurities | 0.13 | |
| **Excipients** | Croscarmellose sodium, lactose monohydrate, magnesium stearate, microcrystalline cellulose, povidone and sodium lauryl sulfate | | Sodium starch glycolate, lactose monohydrate, microcrystalline cellulose, hydroxypropyl cellulose, colloidal silicon dioxide, magnesium stearate, Opadry white-85F18422 |
| ⁽¹⁾ **EZT** (ezetimibe) | | | |

Based on the reference product labeling and relevant literature, Table 4 lists the predicted composition of Zetia (10mg). The composition of bempedoic acid 180 mg tablet, provided by Esperion, is detailed in Table 5.

**Table 4: Predicted Composition of Zetia 10 mg Tablet**

| **Sr. No.** | **Ingredients** | **mg/tablet** |
|---|---|---|
| 01 | Ezetimibe | 10 |
| 02 | Lactose monohydrate | 50-60 |
| 03 | Microcrystalline cellulose | 10-30 |
| 04 | Povidone | 3-5 |
| 05 | Croscarmellose sodium | 6-10 |
| 06 | Sodium lauryl sulfate | 1-3 |
| 07 | Magnesium stearate | 1 |
| **Total weight of Zetia** | | **100** |

**Table 5: Composition of Bempedoic Acid 180 mg Tablet**

| **Sr. No.** | **Ingredients** | **mg/tablet** |
|---|---|---|
| 01 | Bempedoic acid | 180 |
| 02 | Microcrystalline cellulose | 51 |
| 03 | Lactose | 30 |
| 04 | Sodium starch glycolate | 21 |
| 05 | Hydroxypropyl cellulose | 12 |
| 06 | Colloidal silicon dioxide | 1.5 |
| 07 | Magnesium stearate | 4.5 |
| 08 | Opadry white (85F18422) | 9 |
| **Total weight of film coated bempedoic acid tablet** | | **309** |

### Fixed Dose Combination (FDC) Product

The quality target product profile (QTPP) is a prospective summary of the characteristics of a drug product that ideally will be achieved to ensure the desired quality, taking into account safety and efficacy of the drug product. The QTPP is an essential element of a Quality by Design (QbD) approach and forms the basis of design of the drug product. The QTPP set for the FDC product is as listed in Table 6.

**Table 6: Quality Target Product Profile for FDC**

| **QTPP Element** | **Target** | **Justification** |
|---|---|---|
| **Dosage form** | Tablet | Tablet is the most preferred and convenient dosage form for administration to patient. |
| **Route of administration** | Oral | It is the most preferred route of administration to achieve desired efficacy and safety targets. |
| **Dosage strength** | 10 mg ezetimibe + 180 mg Bempedoic acid | Fixed dose combination for effective lowering of lipid levels. |
| **Pharmacokinetics** | Bioequivalent to individual reference drug product RLD | Bioequivalence requirement to both individual reference products. Need to ensure rapid onset of action and efficiency. |
| **Stability** | At least 24-month shelf-life at room temperature | Needed for commercialization. Equivalent to or better than reference product. |
| **Drug product quality attributes** | Physical attributes | Pharmaceutical equivalence requirement. Meeting the same compendia or other applicable (quality) standards (i.e., identity, assay, purity, and quality) |
| | Identification | |
| | Assay | |
| | Content uniformity | |
| | Dissolution | |
| | Degradation products | |
| | Residual solvents | |
| | Water content | |
| | Microbial limits | |
| **Container closure system** | HDPE bottles with Child Resistant (CR) Caps with desiccant | Needed to achieve the target shelf-life and to ensure integrity during shipping for safety and commercial requirements. |
| **Administration/ concurrence with labeling** | To be determined based on outcome of the PK studies | For effectiveness of the proposed dosage form. |

Table 7 summarizes the quality attributes of ezetimibe (10 mg) and bempedoic acid (180 mg) FDC and indicates which attributes were classified as critical quality attributes (CQAs). The CQAs that have the potential to be impacted by the formulation and/or process variables were investigated during development studies. CQAs which are unlikely to be impacted by formulation and/or process variables were not be studied. However, these CQAs are still target elements of the QTPP and are ensured through a good pharmaceutical quality system and the control strategy.

**Table 7: FDC product CQAs**

| **Quality Attributes of the Drug Product** | | **Target** | **Is this a CQA?** | **Justification** |
|---|---|---|---|---|
| | Appearanc e | Color and shape acceptable to the patient. No visual tablet defects observed | No | Color, shape and appearance are not directly linked to safety and efficacy. |
| | | | | Therefore, they are not critical. The target is set to ensure patient acceptability. |
| Physical Attributes | Odor | No unpleasant odor | No | In general, a noticeable odor is not directly linked to safety and efficacy, but odor can affect patient acceptability. For this product, neither the drug substance nor the excipients have an unpleasant odor. |
| | Size | Similar or acceptably greater than current product | No | For ease of swallowing and patient acceptance. |
| | Friability | NMT 1.0% w/w | No | Friability is a routine test per compendia requirements for tablets. |
| | | | | A target of NMT 1.0% w/w of mean weight loss assures a low impact on patient safety and efficacy and minimizes customer complaints. |
| Identification | | Positive for ezetimibe and bempedoic acid | Yes | Identification is critical to safety and efficacy. This CQA can be effectively controlled by the quality management system and will be monitored at drug product release. This CQA will not be studied. |
| Assay | | 100% w/w of label claim of both actives | Yes | Assay variability can affect safety and efficacy. Process variables may affect the assay of the drug product. Hence, assay will be evaluated throughout the development. |
| Content Uniformity (CU) | | Conforms to USP <905> Uniformity of Dosage Units | Yes | Variability in content uniformity will affect safety and efficacy. Both formulation and process variables may impact content uniformity. |
| | | | | Hence, this CQA will be evaluated. |
| Dissolution | | NLT 80% (Q) at 30 minutes for both ezetimibe and bempedoic acid | Yes | Failure to meet the dissolution specification can impact bioavailability. Formulation and process variables both affect the dissolution profile. This CQA will be investigated throughout development |
| Degradation Products | | Specified impurities : NMT 0.2% Any unidentified impurity: NMT 0.2% Total impurities: NMT 1.0% | Yes | Impurities will be monitored based on ICH identification and qualification threshold. This CQA will be investigated throughout formulation development and stability evaluation. |
| Residual Solvents | | USP <467> option 1 | Yes | Residual solvents can impact safety. |
| | | | | However, no solvent is used in the drug product manufacturing process and the drug product complies with USP <467>option 1. Therefore, formulation and process variables are unlikely to impact this CQA. |
| Microbial enumeration test | | Complies with USP <61> and USP <62> | Yes | Non-compliance with microbial limits will impact patient safety. It will be controlled during the manufacturing process. Therefore, this CQA will not be discussed in detail. |
| | | | | |

### Dissolution Method

Dissolution method development for bempedoic acid and ezetimibe FDC product. Table 8 shows the dissolution method used to measure drug release from the FDC product. Drug release profiles for both actives were estimated using common chromatographic conditions with different injection volumes.

**Table 8: Dissolution Method for QC Testing of Individual Reference Products**

| **Dissolution conditions** | **Ezetimibe Tablets** | **Bempedoic Acid Tablets** |
|---|---|---|
| | **(Per USFDA data base)** | **Information** |
| Apparatus | USP Apparatus-II | USP Apparatus-II |
| Rotation speed (rpm) | 50 | 50 |
| Volume (mL) | 500 | 900 |
| Dissolution time | 10, 20, 30 and 45 minutes | 10, 20, 30, 45 min, and infinity time point (additional 15 min at 250 RPM) |
| Dissolution media | S in 0.05M acetate buffer, pH 4.5 | 50 mM phosphate buffer, pH 6.8 |

### Method Development of Ezetimibe Discriminatory Dissolution Method

Ezetimibe exhibits poor aqueous solubility (insoluble in aqueous media at all pH); hence incorporation of SLS in the dissolution media is necessary.

QC dissolution medium, 0.45% SLS in 0.05 M acetate buffer, pH 4.5 yields more than 85% of drug release within 15 minutes. Reduced concentrations of SLS and variable volumes of dissolution media were evaluated to identify suitable discrimination for dissolution of reference product.

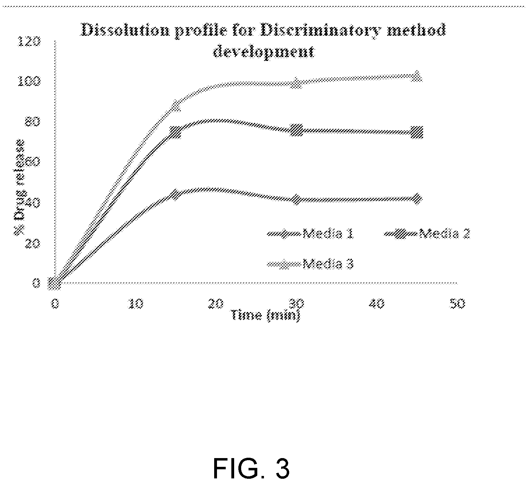

Table 9 and Figure 3 show the dissolution data for reference product combinations at various dissolution conditions.

**Table 9: Dissolution Profile of the Combined Reference Products with Different Dissolution Media**

| **Product** | **Bempedoic Acid +Zetia Reference Product** | | |
|---|---|---|---|
| **Media** | **Medium 1: 500 mL; 0.1% SLS in acetate buffer, pH 4.5** | **Medium 2: 900 mL; 0.1% SLS in acetate buffer, pH 4.5** | **Medium 3: 900 mL; 0.2% SLS in acetate buffer pH 4.5** |
| Time (min) | % Drug release (ezetimibe) | | |
| 15 | 44.0 | 74.7 | 87.8 |
| 30 | 41.5 | 75.6 | 99.3 |
| 45 | 4.20 | 74.6 | 102.9 |

Based on the obtained dissolution profiles, Medium 2 (900 mL acetate buffer, pH 4.5 with 0.1% SLS) was proposed to be discriminatory medium. Media 1 and 3 were not selected because Medium 3 (0.2% SLS) shows dose dumping (as was also observed in the QC release media) and Medium 1 (reduced volume of media) showed inadequate sink conditions to release complete drug.

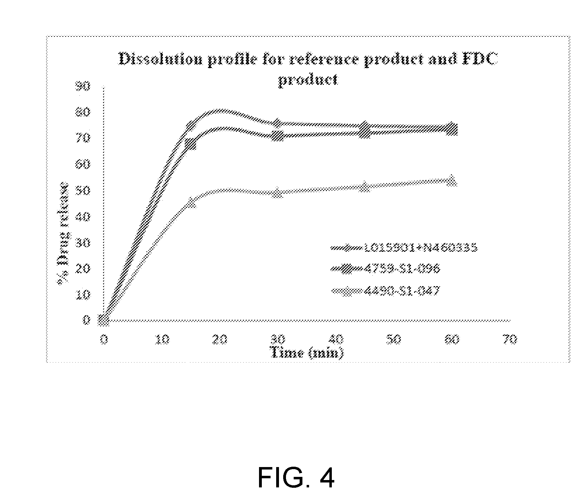

The FDC prototype formulation (Batch no: 4759-S1-096) shows comparable release to the combined reference product (Zetia (10 mg) + bempedoic acid tablet (180 mg)) in discriminatory dissolution media and the discriminatory power of the method is demonstrated in the example shown in Table 10 and Figure 4.

To understand the discriminatory power of the dissolution media, the dissolution profiles of the following two batches was monitored:
- Batch No: 4759-S1-096 - Homogenized ezetimibe with top spray granulation
- Batch No: 4490-S1-047 - Ezetimibe mixed with excipients (diluents & superdisintegrant) followed by wet granulation.

The batch with homogenized API, Batch No: 4759-S1-096, was expected to have a faster release profile compared to the other batch where ezetimibe was not homogenized. Table 10 shows the results of a dissolution comparison with Ezetimibe tablet (10 mg). The dissolution condition was as follows: 0.1% SLS in 0.05 M acetate buffer, pH 4.5. USP Apparatus-II, 50 rpm, 900 mL.

**Table 10: Evaluation of Discriminatory Power of the Selected Dissolution Medium**

| **Product** | **Zetia (10 mg) + Bempedoic acid (180 mg) tablet** (Reference Product) | **FDC Product** | **FDC Product** |
|---|---|---|---|
| **Batch /Lot No.** | L015901+N460335 | 4759-S1-096 | 4490-S1-047 |
| **Time (min.)** | **% Drug Release (ezetimibe)** | | |
| 15 | 74.7 | 67.7 | 45.5 |
| 30 | 75.6 | 70.7 | 49.2 |
| 45 | 74.6 | 71.9 | 51.5 |
| 60 | 74.3 | 73.3 | 54.0 |

The discriminatory dissolution media reflected the difference in processing for ezetimibe granulation and correlated with the expected dissolution behavior. The discriminatory power of the selected media was confirmed. The dissolution profile of Zetia (10 mg) + bempedoic acid (180 mg) (reference product) was found to be comparable with the test FDC product (batch no: 4759-S1-096).

### Method Development of Discriminatory Dissolution Method for Bempedoic Acid

The QC release medium, (phosphate buffer, pH 6.8) showed dose dumping (~ 90% in 15 minutes). Optimization of the surfactant concentration (0.1% to 0.45%) was performed with 1000 mL of different dissolution media using USP Apparatus II at 50 rpm.

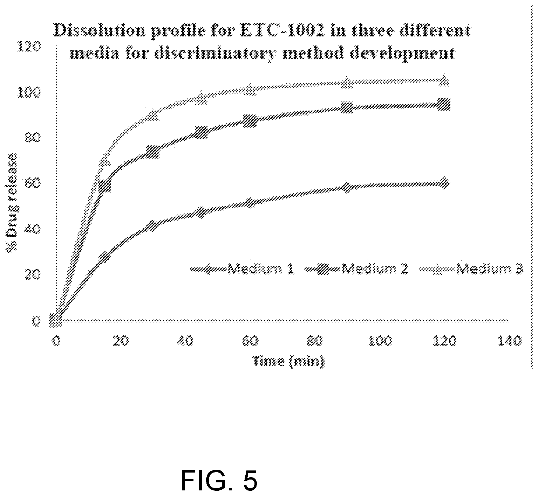

The dissolution was performed on the Zetia (10mg) + bempedoic acid (180mg) reference product and the dissolution data is shown in Table 11 and Figure 5.

**Table 11: Method Development Data for Development of Discriminatory Dissolution Method for Bempedoic Acid**

| **Time Points (minutes)** | **Medium 1: 0.1% SLS in acetate buffer, pH 4.5,** | **Medium 2: 0.45% SLS in acetate buffer, pH 4.5** | **Medium 3: 0.2M phosphate buffer, pH 6.8** |
|---|---|---|---|
| | **% Drug Release (Bempedoic acid)** | | |
| 15 | 27.6 | 58.8 | 70.4 |
| 30 | 41.6 | 73.9 | 90.2 |
| 45 | 47.4 | 82.2 | 97.8 |
| 60 | 51.4 | 87.5 | 101.3 |
| 90 | 58.3 | 93.1 | 104.2 |
| 120 | 60.1 | 94.7 | 105.3 |

Media Medium 1 (0.1% SLS in acetate buffer, pH 4.5) showed a slower release profile and complete recovery was not observed within 2 hrs. Medium 3 (0.2 M phosphate buffer, pH 6.8) showed dumping behavior. Medium 2 (0.45% SLS in acetate buffer, pH 4.5) showed a noticeable gradual dissolution profile for bempedoic acid. Therefore, 0.45% SLS in acetate buffer, pH 4.5, 1000 mL, 50 rpm, USP App-II was finalized as the discriminatory dissolution condition.

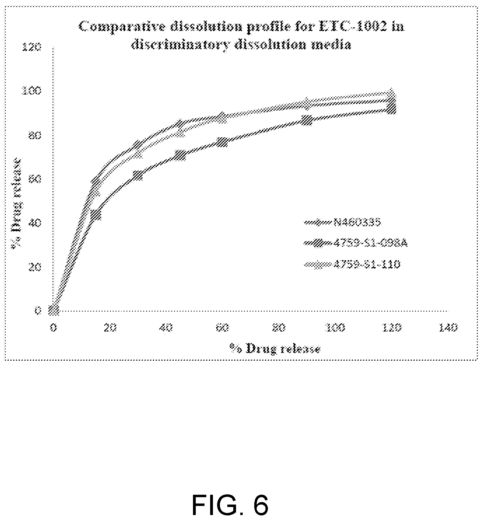

The discriminatory power of the method was demonstrated with slight change in particle size of bempedoic acid in the formulation and is shown in Table 12 and Figure 6.

**Table 12: Comparative Dissolution Profile of Bempedoic Acid Tablet (Reference Product) vs. FDC-Monolayer Tablet with Coarser and Fine Grade Bempedoic Acid**

| **Dissolution comparison for ETC- 1002** | | | |
|---|---|---|---|
| **Dissolution condition: 0.45% SLS in acetate buffer, pH 4.5, 1000 mL, 50 rpm, USP** | | | |
| **Product** | **Bempedoic Acid Tablet** (Reference Product) | **Trial with Coarser Grade Bempedoic Acid** | **Trial with Finer Grade Bempedoic Acid** |
| **PSD of API** | | **% Retained / % passed** | |
| # 60 retained/ passed | | 32.0/68.0 | 11.5/88.5 |

| **Batch /Lot No.** | **N460335** | **4759-S1-098A** | **4759-S1-110** |
|---|---|---|---|
| **Time (min.)** | **% Drug Release (bempedoic acid)** | | |
| 15 | 58.9 | 43.8 | 54.3 |
| 30 | 75.4 | 61.5 | 71.7 |
| 45 | 85.1 | 70.6 | 81.3 |
| 60 | 88.5 | 76.8 | 87.6 |
| 90 | 93.3 | 86.6 | 95.1 |
| 120 | 95.8 | 91.7 | 99.4 |
| R | 96.5 | 94.9 | 101.8 |
| **F2 Value** | | **46.0** | **73.0** |

As expected the batch with coarser grade bempedoic acid (#60 retained/pass = 32/68) showed lower release compared to the batch with finer grade bempedoic acid (#60 retained/pass = 11.5/88.5). The difference in dissolution profile (due to change in particle size of active) reflects the discriminatory power of the selected dissolution condition.

### 5 Drug Substance

Bempedoic acid was obtained from Esperion Therapeutics, Inc. and Ezetimibe was procured from Teva API India Ltd.

### 5.1 Physical Properties

Table 13 gives the physical properties for both APIs.

**Table 13: Physical Properties of Ezetimibe and Bempedoic Acid**

| **Properties** | **Description** | | |
|---|---|---|---|
| | Bempedoic Acid | | Ezetimibe |
| Appearance | White to off white crystalline powder. | | White, crystalline powder. |
| Crystal habit | Hexagonal/Blades | | Agglomerated |
| Hygroscopicity | The absorption/desorption isotherm is consistent with bempedoic acid being non-hygroscopic | | Hygroscopic |
| Bulk density (gm/mL) | 0.310 | | 0.19 |
| Tap density (gm/mL) | 0460 | | 0.27 |
| Compressibility Index (CI) | 32.6 (Very poor) | | 29.6 (Poor) |
| Hausner ration (HR) | 1.48 (Very poor) | | 1.42 (Poor) |
| Flowdex | 18mm bore (55.55) | | 20 mm bore (50) |
| Solubility in Water | Practically insoluble in water. | | Practically insoluble in water. |

| | PSD data: | | |
|---|---|---|---|
| | Sieve No | % Retained | |
| Particle size distribution | #60 | 32.0 | **By Malvern** |
| | #80 | 13.8 | |
| | #120 | 10.4 | |
| | #120 passed | 43.8 | |
| Microscopic photograph (40x) | 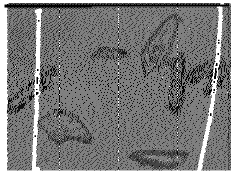 | 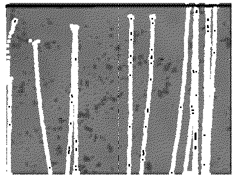 | |

The compressibility indices of 32.6 % and 29.60 % and Hausner's ratios of 1.48 and 1.42 for bempedoic acid and ezetimibe, respectively, suggest very poor flowability of the APIs.

### 5.2 Chemical Properties:

Chemical properties are detailed in Table 14.

**Table 14: Chemical Properties of Ezetimibe and Bempedoic Acid**

| Properties | Description | |
|---|---|---|
| | Bempedoic Acid | Ezetimibe |
| Structural Formula | 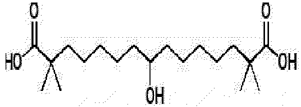 | 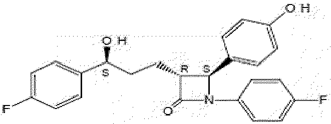 |
| Chemical Name | 8-hydroxy-2,2,14,14-tetramethylpentadecanedioic acid | (3R,4S)-1-(4-fluorophenyl)-3-[(3S)-3- (4-fluorophenyl)-3-hydroxypropyl]-4-(4-hydroxyphenyl) azetidin-2-one. |
| Molecular Formula | C19H36O5 | C24H21F2NO3 |
| Molecular Weight | 344.49 g /mol | 409.4 g /mol |
| Melting Point | 88° C - 91° C | 164° C - 166° C |
| pKa | 4.88, 5.60 (determined by potentiometric titration) | 9.75 (determined by potentiometric titration) |

### 5.2.1 Polymorphism

Ezetimibe drug substance possesses different polymorphic/hydrate forms.
Anhydrous (designated as Form A)
Hydrate form (designated as Form B)

Both polymorphic forms exhibit the same physicochemical properties. For FDC pharmaceutical development, the anhydrous form of ezetimibe (Form A) was used.

Bempedoic acid is a crystalline powder with no evidence of polymorphic formation.

### 5.2.2 Chemical Stability

Forced degradation of bempedoic acid was carried out to study the impurity profile, degradation pathway, and to facilitate the development of a stability-indicating method. In addition, knowledge obtained from the forced degradation studies was used during formulation and process design and development to prevent impurities from being generated. The specified stress conditions were intended to achieve 5 - 20% degradation. The stressed samples were compared to the unstressed sample (control).

Forced degradation conditions and results for bempedoic acid are listed in Table 15.

**Table 15: Summary of Forced Degradation Data for Bempedoic Acid**

| Exposed to (1) | Assay % | % Degradation | | | | | | | | |
|---|---|---|---|---|---|---|---|---|---|---|
| | | RRT 0.94 | RRT 0.97 | RRT 1.03 | RRT 1.04 | RRT 1.25 | RRT 1.28 | RRT 1.95 | RRT 2.29 | Total impurities |
| Non stressed bempedoic acid | 99.0 | -- | -- | -- | -- | -- | -- | -- | -- | -- |
| HCl | 98.7 | -- | -- | 0.06 | -- | -- | 1.03 | -- | -- | 1.09 |
| NaOH | 117.3( 2) | -- | -- | -- | 0.22 | -- | -- | -- | 0.85 | 1.07 |
| H2O2 | 100.4 | -- | -- | -- | -- | -- | -- | -- | | -- |
| Thermal, solution 105 □ □ C | 97.7 | 0.07 | -- | -- | -- | -- | -- | 2.09 | 0.56 | 2.72 |
| Thermal, solid state 105° C | 95.8 | -- | 0.08 | -- | 0.20 | 0.06 | -- | 2.06 | -- | 2.40 |
| Photo, solid state | 99.2 | -- | -- | -- | -- | -- | -- | -- | -- | -- |

| | | | | | | | | | | |
|---|---|---|---|---|---|---|---|---|---|---|
| (1)Appropriate control samples were prepared for each stressor but are not shown in above table. (2) w/w% bempedoic acid in NaOH result differs from expected value of approximately 100% due to non-quantitative neutralization prior to injection | | | | | | | | | | |

No significant change was observed in the purity profile of Bempedoic acid during the stress test study. This indicates that the API is stable at all studied conditions.

The summary of forced degradation data for ezetimibe are presented in Table 16.

**Table 16: Summary of Forced Degradation Data for Ezetimibe**

| **Exposed to** | **Assay (%)** | **% Degradation** | | | |
|---|---|---|---|---|---|
| | | **EZT-Amide** | **EZT Cyclic ether** | **EZT-Keto** | **Total (unspecified and specified)** |
| Non stressed ezetimibe | 100.0 | ND | 0.07 | ND | 0.12 |
| 5.0 mL stock solution + 1mL 1N HCl at RT for 24 hrs | 97.9 | ND | 0.09 | ND | 0.83 |
| 5.0 mL stock solution + 0.1 mL 1N NaOH at RT for 30 min | 83.1 | 1.06 | 9.23 | ND | 14.66 |
| 5.0 mL stock solution + 1mL H2O2 30% at RT for 24 hrs | 97.0 | ND | 0.75 | ND | 0.91 |
| 5.0 mL stock solution in Sun cabinet 765W/m2 at 35°C for 15 hrs | 95.0 | ND | 0.40 | 0.05 | 1.57 |
| 5.0 mL stock solution + Heating at 80°C for 30 min | 93.1 | ND | 4.81 | ND | 5.67 |
| 21.41 mg of solid + Heating at 80°C for 24 hrs | 96.3 | ND | 0.09 | ND | 0.14 |
| 18.48 mg of solid in Sun cabinet 765 W/m2at 35°C for 24 hrs | 98.0 | ND | 0.06 | ND | 0.22 |

It has been observed that ezetimibe is relatively unstable particularly upon exposure to alkali (NaOH) and peroxide (H2O2). The main degradation product identified is EZT cyclic ether. In the solid form however it is stable to heat and photo irradiation. Therefore, ezetimibe is classified as sensitive to alkali and peroxide.

### 5.3 Biological Properties

Biological properties are given in Table 17.

**Table 17: Biological Properties of Bempedoic Acid and Ezetimibe**

| Properties | Description | |
|---|---|---|
| | Bempedoic Acid | Ezetimibe |
| Category | Low-density lipoprotein cholesterol (LDL-C) lowering agent | Cholesterol absorption inhibitors |
| Maximum recommended dose | 180 mg, once a day | 10 mg, once a day with or without food. |
| BCS class | BCS class II | BCS class II |
| Log partition coefficient (log p) | Octanol / water : 4.328 Octanol / pH 3.5 buffer :4.382 | n-octanol: 0.1N HCl:4.52 |
| | | n-octanol : pH 7 : 4.51 |

### 5.4 Risk Assessment of Drug Substance Attributes

A risk assessment of the drug substance attributes was performed to evaluate the impact of each attribute on the drug product CQAs. The relative risk ranking system used throughout the pharmaceutical development is summarized in Table 18. The outcome of the assessment and the accompanying justifications are provided in Table 19, Table 20, Table 21, and Table 22. The relative risk that each drug substance attribute was ranked as high, medium, or low. Those attributes that could have a high impact on the drug product CQAs warranted further investigation whereas those attributes that had low impact on the drug product CQAs required no further investigation.

**Table 18: Overview of relative risk ranking system**

| | |
|---|---|
| ***Low*** | Broadly acceptable risk. No further investigation is needed. |
| ***Medium*** | Risk is acceptable. Further investigation may be needed in order to reduce the risk. |
| ***High*** | Risk is unacceptable. Further investigation is needed to reduce the risk. |

**Table 19: Risk assessment for Bempedoic Acid**

| **Drug product CQA** | **Drug Substance Attributes** | | | | | | | | |
|---|---|---|---|---|---|---|---|---|---|
| | **Solid state form** | **Particle size distribution** | **Solubility** | **Moisture content** | **Chemical stability** | **Flow properties** | **Hygroscopicity** | **Total impurity** | **Residual solvent** |
| **Assay** | Low | Low | Low | Low | Low | Medium | Low | Low | Low |
| **Content uniformity** | Low | Low | Low | Low | Low | Medium | Low | Low | Low |
| **Dissolution** | Low | Medium | Medium | Low | Low | Low | Low | Low | Low |
| **Degradation product** | Low | Low | Low | Low | Low | Low | Low | Medium | Low |

**Table 20: Risk Assessment Justification for Impact of Drug Substance Attributes of Bempedoic Acid on Drug Products CQA**

| **Drug Substance Attributes** | **Drug product CQA** | **Justification** |
|---|---|---|
| Solid state form | Assay | Drug substance solid state form does not affect tablet assay and CU. The risk is low. |
| | Content uniformity | |
| | Dissolution | Bempedoic acid does not have any polymorphic forms. The solid state of bempedoic acid has no impact on dissolution. The risk is low. |
| | Degradation product | The solid form of BEMPEDOIC ACID used in the development is stable; it will not contribute to degradation product. The risk is low. |
| PSD | Assay | Bempedoic acid quantity in the formulation is more than 40%. The risk is low. |
| | Content uniformity | |
| | Dissolution | Bempedoic acid has low solubility. PSD may affect solubility and thus dissolution. The risk is medium. |
| | Degradation product | Particle size does not impact the degradation products. The risk is low. |
| Solubility | Assay | Solubility does not have an impact on assay & content uniformity. The risk is low. |
| | Content uniformity | |
| | Dissolution | Bempedoic acid has low solubility which may impact dissolution. The risk is medium. |
| | Degradation product | Solubility does not affect tablet degradation products. The risk is low. |
| Moisture content | Assay | Bempedoic acid is non-hygroscopic. Moisture content of the drug substances does not impact assay, CU, dissolution, and degradation products. The risk is low. |
| | Content uniformity | |
| | Dissolution | |
| | Degradation product | |
| Chemical stability | Assay | Bempedoic acid is chemically stable. The impact to chemical stability of drug substance on assay, CU, dissolution and degradation product is minimal. The risk is low. |
| | Content uniformity | |
| | Dissolution | |
| | Degradation product | |
| Flow Properties | Assay | Bempedoic acid has poor flow properties which can have impact on assay and CU. The risk is medium. |
| | Content uniformity | |
| | Dissolution | The flowability of the drug substance is unlikely to affect its degradation pathway or solubility. The risk is low. |
| | Degradation product | |
| Hygroscopicity | Assay | Bempedoic acid is non hygroscopic hence it is unlikely to impact Assay, Content uniformity, Dissolution, and Degradation product of drug product. The risk is low. |
| | Content uniformity | |
| | Dissolution | |
| | Degradation product | |
| Total impurities | Assay | Total impurities are controlled in the drug substance specification (NMT 2.0%). Impurity limits comply with ICH Q3A recommendations. Within this range, process impurities are unlikely to impact assay, CU and dissolution. The risk is low. |
| | Content uniformity | |
| | Dissolution | |
| | Degradation product | Process impurities that are potential degradants may increase over time. The risk is medium. |
| Residual solvents | Assay | Residual solvents are controlled in the drug substance specification and comply with USP <467>. At controlled levels, residual solvents are unlikely to impact assay, CU, dissolution & degradation. The risk is low. |
| | Content uniformity | |
| | Dissolution | |
| | Degradation product | |

**Table 21: Risk Assessment for Ezetimibe**

| **Drug product CQA** | **Drug Substance Attributes for Ezetimibe** | | | | | | | | |
|---|---|---|---|---|---|---|---|---|---|
| | **Solid state form** | **PSD** | **Solubility** | **Moisture content** | **Chemical stability** | **Flow properties** | **Hygroscopicity** | **Process impurity** | **Residual solvent** |
| **Assay** | Low | Medium | Low | Low | Low | Medium | Low | Low | Low |
| **Content uniformity** | Low | Medium | Low | Low | Low | Medium | Low | Low | Low |
| **Dissolution** | Low | High | Medium | Low | Low | Low | Low | Low | Low |
| **Degradation product** | Low | Low | Low | Low | Medium | Low | Low | Medium | Low |

**Table 22: Risk Assessment Justification for Impact of Drug Substance Attributes on the Drug Product CQAs**

| **Drug Substance Attributes** | **Drug Product CQA** | **Justification** |
|---|---|---|
| Solid state form | Assay | The anhydrous form of ezetimibe was used for the development. Both anhydrous and hydrate forms show similar physical and chemical properties which will not affect Assay, Content uniformity, Dissolution, Degradation product. The risk is low. |
| | Content uniformity | |
| | Dissolution | |
| | Degradation product | |
| PSD | Assay | A small particle size and a wide PSD may adversely impact blend flowability. In extreme cases, poor flowability may impact assay. The risk is medium. |
| | Content uniformity | Particle size distribution has a direct impact on drug substance flowability and ultimately on CU. Improper mixing with excipients may lead to problems in assay and content uniformity. The risk is medium. |
| | Dissolution | Drug substance has low solubility. PSD may affect solubility and hence the dissolution. The risk is high. |
| | Degradation product | Particle size is unlikely to impact the degradation products. The risk is low. |
| Solubility | Assay | Solubility is unlikely to have an impact on assay & content uniformity. The risk is low. |
| | Content uniformity | |
| | Dissolution | Ezetimibe exhibited low solubility across physiological pH range. Drug substance solubility does impact dissolution. The risk is medium. |
| | Degradation product | Solubility is unlikely to affect tablet degradation products. Thus, the risk is low. |
| Moisture content | Assay | Moisture is controlled in the drug substance specification (NMT 1.5%). Thus, it is unlikely to impact assay, content uniformity and dissolution. The risk is low. |
| | Content uniformity | |
| | Dissolution | |
| | Degradation product | |
| Chemical stability | Assay | Total impurities are controlled in the drug substance specification (NMT 0.5%). Impurity limits comply with ICH Q3A recommendations. Within this range, process impurities are unlikely to impact assay, CU and dissolution. The risk is low. |
| | Content uniformity | |
| | Dissolution | |
| | Degradation product | Impurity of drug substance may impact the formulation impurity profile. The risk is medium. |
| Flow properties | Assay | Ezetimibe has poor flow properties which can have impact on assay and CU. The risk is medium. |
| | Content uniformity | |
| | Dissolution | The flowability of the drug substance is unlikely to affect its degradation pathway or solubility. The risk is low. |
| | Degradation product | |
| Hygroscopicity | Assay | Ezetimibe is hygroscopic; however, in the granules LOD is controlled with a limit of NMT 2.0% w/w. Further, the HDPE packed with desiccant ensures the physical stability. The risk is low. |
| | Content uniformity | |
| | Dissolution | |
| | Degradation product | |
| Process impurities | Assay | Total impurities are controlled in the drug substance specification (NMT 0.15%). Impurity limits comply with ICH Q3A recommendations. Within this range, process impurities are unlikely to impact assay, CU, and dissolution. The risk is low. |
| | Content uniformity | |
| | Dissolution | |
| | Degradation product | Process impurities may increase upon stability. The risk is medium. |
| Residual solvents | Assay | Residual solvents are controlled in the drug substance specification and comply with USP <467>. At controlled levels, residual solvents are unlikely to impact assay, CU, dissolution & degradation. The risk is low. |
| | Content uniformity | |
| | Dissolution | |
| | Degradation product | |

### 5.5 Drug-Drug Interaction

The fixed dose combination product under development is a novel combination of two drug substances and therefore determination of compatibility of the actives with each other was considered to be critical. The compatibility of the actives was assessed through HPLC analysis of binary mixtures of the drug substances at a ratio 1:18 (ezetimibe: bempedoic acid) in the solid state. Samples were stored at 60°C and 40°C / 75%RH in both open and closed containers for 2 weeks and 4 weeks respectively. Degradation products were evaluated for open (stressed) samples of accelerated condition (40°C / 75% RH) and 60°C. Assay for the samples was also carried out. Results are summarized in Table 23.

**Table 23: Drug-Drug Compatibility Study Results**

| Samples | Condition | EZT cyclic ether | RRS isomer | EZT 21 (Keto impurity) | SMUI | Total impurities | Assay | |
|---|---|---|---|---|---|---|---|---|
| Ezetimibe | Initial | ND | ND | 0.03 | 0.03 | 0.06 | 99.3 | |
| | 2W 60° | 0.12 | ND | 0.04 | 0.17 | 0.33 | 97.6 | |
| | 4W 40°C / 75% RH Open | 0.90 | ND | 0.38 | 0.19 | 2.34 | 99.5 | |
| Bempedoic acid | Initial | No unspecified impurity generated | | | | | 101.7 | |
| | 2W 60° | | | | | | 100.2 | |
| | 4W 40°C / 75% | | | | | | 103.7 | |

| | | | | | | | EZT | Bempedoic Acid |
|---|---|---|---|---|---|---|---|---|
| Ezetimibe + Bempedoic acid | Initial | ND | ND | ND | ND | ND | 100.0 | 101.3 |
| | 2W 60° | ND | ND | 0.05 | ND | 0.05 | 103.8 | 97.6 |
| | 4W 40°C / 75% | ND | ND | 0.04 | 0.13 | 0.17 | 99.0 | 98.2 |

There was no significant increase in any of the impurities for each of the actives in presence of each other under accelerated conditions and the assay was found to be within the acceptable limits. Therefore, bempedoic acid and ezetimibe can be considered to be chemically compatible.

### 5.6 Excipients

The excipients used in the combination drug product were selected based on the excipients used in the individual reference products, excipient compatibility studies, and prior use in approved drug products. The drug - excipient compatibility studied combined both APIs with selected excipients.

### 5.6.1 Drug - Excipient Compatibility Study

Excipients-drug substance compatibility was assessed using HPLC analysis of ternary mixtures of excipients and both APIs together at a required ratio in the solid state. Samples were stored at 60°C and 40°C / 75%RH in both open and closed containers for 2 weeks and 4 weeks respectively. Common excipients functioning as filler, disintegrant, binder, and lubricant were evaluated. Degradant level (related substance) for each API was assessed using HPLC analysis to quantify the degradations in case of any incompatibility. Degradation products were evaluated for open (stress) samples at accelerated conditions (40°C / 75% RH) and 60°C. Samples held closed at accelerated condition (40°C / 75% RH) were evaluated when there was a significant increase in degradation observed in the open (stress) conditions. Assay for the samples was also carried out. Results are summarized in Table 24.

Table 25 describes the compatibility study of both actives together with the excipients used in the formulation.

**Table 24: Batches for drug - excipient compatibility study**

| **Sr. No.** | **Ingredients** | **Batch No.** | **Ratio** |
|---|---|---|---|
| 1 | (Ezetimibe + Bempedoic acid)+ Pharmatose 200M | 4490-CO-014-O | 1:1 |
| 2 | (Ezetimibe + Bempedoic acid)+ Avicel PH- 102 | 4490-CO-014-P | 1:1 |
| 3 | (Ezetimibe + Bempedoic acid)+ Kollidon 30 | 4490-CO-014-Q | 1:0.5 |
| 4 | (Ezetimibe + Bempedoic acid)+ HPC-L | 4490-CO-014-R | 1:0.5 |
| 5 | (Ezetimibe + Bempedoic acid)+ Kolliphor SLS Fine | 4490-CO-014-T | 1:0.5 |
| 6 | (Ezetimibe + Bempedoic acid)+ Primojel | 4490-CO-014-V | 1:0.5 |
| 7 | (Ezetimibe + Bempedoic acid) +Aerosil 200P | 4490-CO-014-W | 1:0.1 |
| 8 | (Ezetimibe + Bempedoic acid) + magnesium stearate | 4490-CO-014-X | 1:0.1 |
| 9 | (Ezetimibe + Bempedoic acid) + Opadry white (85F18422) | 4490-CO-014-Z | 1:0.25 |

**Table 25: Drug-Excipient Compatibility Study Results**

| **Samples** | **Condition** | **EZT cyclic ether** | **RRS isomer** | **EZT 21 (Keto impurity)** | **SMUI** | **Total impurities** | **Assay** | |
|---|---|---|---|---|---|---|---|---|
| | | | | | | | **EZT** | **Bempedoic Acid** |
| Ezetimibe + Bempedoic acid + Pharmatose 200M ) | Initial | ND | ND | ND | ND | ND | 103.1 | 98.2 |
| | 2W 60° Open | ND | ND | 0.04 | ND | 0.04 | 97.0 | 96.8 |
| | 4W 40°C / 75%RH Open | ND | ND | 0.05 | 0.04 | 0.09 | 97.8 | 99.9 |
| Ezetimibe + Bempedoic acid + Avicel PH-102 | Initial | ND | ND | ND | ND | ND | 95.2 | 101.6 |
| | 2W 60° Open | ND | ND | 0.04 | 0.03 | 0.07 | 97.4 | 96.3 |
| | 4W 40°C / 75%RH Open | ND | ND | 0.04 | 0.02 | 0.06 | 99.0 | 99.4 |
| Ezetimibe + Bempedoic acid + Kollidon 30 | Initial | ND | ND | 0.03 | ND | 0.03 | 98.0 | 98.6 |
| | 2W 60° Open | ND | ND | 0.08 | ND | 0.08 | 99.9 | 95.0 |
| | 4W 40°C / 75%RH Open | 0.13 | ND | 0.16 | 0.02 | 0.35 | 99.3 | 95.7 |
| Ezetimibe + Bempedoic acid + HPC-L | Initial | ND | ND | 0.03 | ND | 0.03 | 101.1 | 95.9 |
| | 2W 60° Open | 0.11 | ND | 0.15 | 0.10 | 0.039 | 99.4 | 98.2 |
| | 4W 40°C / 75%RH Open | 0.34 | ND | 0.06 | 0.2 | 0.46 | 101.1 | 96.4 |
| | 4W 40°C / 75%RH closed | ND | ND | ND | ND | 0.02 | Not done | |
| Ezetimibe + Bempedoic acid + Kolliphor SLS Fine | Initial | ND | ND | 0.03 | ND | 0.03 | 99.1 | 101.6 |
| | 2W 60° Open | ND | ND | 0.04 | ND | 0.04 | 96.5 | 100.7 |
| | 4W 40°C / 75%RH Open | ND | ND | 0.04 | ND | 0.04 | 97.4 | 99.5 |
| Ezetimibe + Bempedoic acid + Primojel (Batch No 4490- CO-0 14-V) | Initial | ND | ND | 0.03 | ND | 0.03 | 97.3 | 102.3 |
| | 2W 60° Open | ND | ND | 0.05 | ND | 0.05 | 98.1 | 101.9 |
| | 4W 40°C /75%RH Open | ND | ND | 0.04 | ND | 0.04 | 98.3 | 99.8 |
| Ezetimibe + Bempedoic acid + Aerosil 200P | Initial | ND | ND | ND | ND | ND | 95.9 | 101.1 |
| | 2W 60° Open | ND | ND | 0.07 | 0.04 | 0.11 | 104.4 | 98.5 |
| | 4W 40°C / 75%RH Open | ND | ND | 0.04 | 0.04 | 0.08 | 97.7 | 103.6 |
| Ezetimibe + Bempedoic acid + Magnesium stearate | Initial | ND | ND | ND | ND | ND | 99.8 | 101.1 |
| | 2W 60° Open | ND | ND | 0.06 | ND | 0.06 | 98.7 | 99.5 |
| | 4W 40°C / 75%RH Open | ND | ND | 0.05 | 0.02 | 0.07 | 99.1 | 100.9 |
| Ezetimibe + Bempedoic acid + | Initial | ND | ND | 0.03 | ND | 0.03 | 102.9 | 104.8 |
| | 2 W 60°C Open | 9.05 | ND | 0.13 | 1.38 | 11.06 | 91.7 | 100.7 |
| Opadry white (85F 18422) | 4W 40°C / 75%RH Open | 6.26 | ND | 6.32 | 0.95 | 7.66 | 91.8 | 101.2 |
| | 4W 40°C / 75%RH Closed | 6.27 | ND | 0.08 | 0.11 | 0.29 | - | |

No significant change in appearance was observed for Bempedoic acid; however an increase in cyclic ether impurity was observed for open Ezetimibe at 40°C / 75% RH. The combination of high temperature and humidity could have triggered this degradation. However, in the presence of Bempedoic acid and excipient, Ezetimibe is diluted and the impact of heat and humidity could be reduced.

An increase in the Ezetimibe cyclic ether was observed when combined with HPC-L in an open container maintained for 4W at 40°C/75% RH. However, this drug - excipient combination was found to be compatible at 4W 40°C / 75% RH when in a closed container. HPC-L is used in the granulation of Bempedoic acid and is not in direct contact with Ezetimibe; therefore, the excipient was selected in the final formulation.

Assay values of both Ezetimibe and Bempedoic acid were comparable to the initial samples, except for Opadry white where a drop in the assay value of Ezetimibe was observed along with an increase in the impurity levels. With increase in impurities at all conditions, the excipient, Opadry white (85F 18422), was found to be incompatible. It is important to note that the reference product for Bempedoic acid mono product is a coated tablet whereas the reference product, Zetia, is uncoated tablet. The target for the fixed dose combination is a coated tablet. Therefore, an additional compatibility study with another Opadry system, without polyethylene glycol and polyvinyl alcohol (main component of studied Opadry) was proposed.

### 5.6.2 Selection of Excipient Grade

Based on the results of the drug-excipient compatibility studies, excipients identical to the reference product formulations were selected for the development of the fixed dose combination product. The levels of excipients to be used in the formulation were studied in subsequent formulation development studies.

### Lactose Monohydrate (Pharmatose^{®} 200M):

Lactose monohydrate is commonly used as filler. Usually, fine grades of lactose are used in the preparation of tablets using a wet-granulation method. Pharmatose^{®} 200M, a lactose monohydrate, from DFE Pharma was selected. Particle size distribution data of Pharmatose 200M shows more than 90% particles are less than 100 µm size and a bulk density of 0.56 g/cm3.

### Microcrystalline Cellulose (Avicel^{®} PH-102):

Microcrystalline cellulose is widely used as filler in direct compression and roller compaction. It undergoes plastic deformation during compaction since it is fibrous and ductile. Microcrystalline cellulose (Avicel^{®} PH-102) is used in the current bempedoic acid tablet formulation as a diluent with larger particle size (100 µm) which helps to provide better blend flow properties. The moisture content is 3.0 - 5.0 % and the bulk density is 0.28 - 0.33 g/cc.

### Hydroxypropyl Cellulose (HPC-L):

Hydroxypropyl cellulose is a partially-substituted poly (hydroxypropyl) ether of cellulose. Hydroxypropyl cellulose is commercially available in a number of different grades that have various solution viscosities. The grade to be used in the formulation is the regular grade fine powder with a range in viscosity range from 6.0 to 10.0 mPa.s, which is mostly used as a binder in tablet dosage form.

### Povidone K30 (Kollidon^{®} 30):

Povidone (Kollidon^{®} 30) is a medium molecular weight grade of povidone with a K value of 27.0 - 32.1. It is versatile and widely used as a binder in tablets and granules. In the current formulation, an aqueous solution of povidone is used as a binder in the top spray granulation process for ezetimibe.

### Sodium Lauryl Sulfate (Kolliphor^{®} SLS Fine):

Sodium lauryl sulfate is an anionic surfactant employed in a wide range of oral pharmaceutical formulations for the dissolution improvement of insoluble drug molecules.

Sodium lauryl sulfate is used as a solubilizer in concentrations greater than critical micelle concentration i.e. > 0.0025%. It is used as wetting agent, effective in both alkaline and acidic conditions. In the current formulation it is used as a dissolution enhancer for ezetimibe.

### Sodium Starch Glycolate (Primojel^{®}):

Sodium starch glycolate is a white or almost white free-flowing very hygroscopic powder. It is widely used in oral pharmaceuticals as a disintegrant in tablet manufacturing. Disintegration occurs by rapid uptake of water followed by rapid and enormous swelling of tablets containing sodium starch glycolate.

The effectiveness of many disintegrants is affected by the presence of hydrophobic excipients, such as lubricants. Increasing the tablet compression pressure appears to have no effect on disintegration time.

### Colloidal Silicon Dioxide (Aerosil^{®} 200P):

Colloidal silicon dioxide (Aerosil^{®} 200 Pharma), a commercial grade of colloidal silicon dioxide manufactured by Evonik, was used as a glidant in the current bempedoic acid tablet formulation.

### Magnesium Stearate:

Magnesium stearate, manufactured by Avantor using a vegetable origin, was used as lubricant in the current formulation. It has particle size specification of 99 to 100% w/w passing through # 325 sieve (ASTM, 45 µm) and LOD < 5.0 % w/w.

### Opadry AMB II 88A180040 White:

Opadry AMB II 88A180040 is a polyvinyl acetate (PVA) based non-functional film coating system with glycerol monocaprylocaprate type 1, sodium lauryl sulfate, titanium dioxide and talc used for the aesthetic appearance of the tablets.

Table 26 and Table 27 summarize the grade excipients selected for the proposed formulation and their IID limits.

**Table 26: IID Limits of Excipients in Proposed FDC Formulations**

| Ingredients | Specification | Current formula (mg/unit) | IID Limit | Route specified in IID listing |
|---|---|---|---|---|
| Ezetimibe | IH | 10.00 | - | - |
| Bempedoic acid | IH | 180.00 | - | - |
| Lactose monohydrate (Pharmatose^{®} 200M) | USP/NF | 75.00 | 587.44 | Tablet, film coated |
| Microcrystalline cellulose (Avicel^{®} PH- 102) | NF/Ph. Eur | 60.60 | 563.50 | Tablet, film coated |
| Sodium starch glycolate (Primojel^{®}) | USP/NF/Ph. Eur | 27.0 | 90.00 | Tablet, film coated |
| Povidone K30 (Kollidon^{®} 30) | USP/NF/Ph. Eur | 1.00 | 80.00 | Tablet |
| Sodium lauryl sulfate (Kolliphor^{®} SLS Fine) | USP/NF/Ph. Eur | 2.00 | 12.00 | Tablet, film coated |
| Colloidal silicon dioxide (Aerosil^{®} 200P) | USP/NF/Ph. Eur | 4.00 | 33.00 | Tablet, film coated |
| Hydroxypropyl cellulose (HPC-L) | EP | 12.00 | 52.50 | Tablet, film coated |
| Magnesium stearate | NF | 4.00 | 40.00 | Tablet, coated |
| Total weight of core FDC tablet | | 375.6 | NA | NA |
| Opadry AMB II 88A180040 white | IH | 9.4 | Refer to Table 25 | |
| Total weight of coated FDC tablet | | 385.0 | NA | NA |

**Table 27: Composition of Opadry AMB II 88A180040 White**

| Ingredients | Quality standard | Composition (% w/w) | mg/tablet | IID Limit (mg) | Route specified in IID listing |
|---|---|---|---|---|---|
| Polyvinyl alcohol part. hydrolyzed | USP and Ph.Eur. | 37.0 | 3.48 | 20.0 | Tablet, film coated |
| Talc | USP and Ph.Eur. | 31.0 | 2.91 | 320.75 | Tablet, coated |
| Titanium dioxide | USP and Ph.Eur. | 25.0 | 2.35 | 10.57 | Tablet, |
| Glycerol monocaprylocaprate Type 1 | Ph.Eur. | 4.0 | 0.38 | 62.2 | Capsule, gelatin |
| Sodium lauryl sulfate | NF and Ph.Eur. | 3.0 | 0.28 | 12.0 | Tablet, film coated |

### 6. Drug Product

### 6.1 General Information

**Table 28: Drug product general information**

| | | |
|---|---|---|
| Non-Proprietary Name of Drug substance | : | Bempedoic acid and ezetimibe |
| Dosage Form | : | Immediate release tablets |
| Strength (s) | : | Bempedoic acid - 180 mg and ezetimibe - 10 mg |
| Route of Administration | : | Oral |
| Proposed Indication (s) | : | Treatment of hypercholesterolemia |

### 6.2 Formulation Development

Compatibility studies revealed that both bempedoic acid and ezetimibe were chemically compatible with each other and therefore it was possible to develop a monolayer tablet. However, the individual APIs will be granulated separately for the following reasons.

Ezetimibe exhibits poor aqueous solubility across physiological pH and therefore a surfactant, sodium lauryl sulfate (SLS), was employed in Zetia formulation. The same is preferred in the combination product to achieve bioequivalence. Bempedoic acid, however, despite having pH dependent solubility, does not require a surfactant as established in the mono product already developed by Esperion. Its increased solubility at high pH ensures dissolution and absorption in-vivo.

Bempedoic acid is sticky and has poor flowability. This aspect requires certain process steps and/or excipients that may not be suitable for ezetimibe.

### Formulation options:

Compatibility studies ruled out any potential chemical interaction between the two APIs; however, it may be still possible that some physical interaction may lead to compromised dissolution. Additionally, the presence of SLS in a monolayer formulation may alter the dissolution of bempedoic acid and its absorption. Therefore, it was decided to develop following two formulations:
FDC-Monolayer, with both actives (in the form of separate granules) compressed into a single layer tablet, and
FDC-Bilayer, with both granules compressed into a two layer tablet with actives present in separate layers.

### 6.2.1 Initial Risk Assessment of the Formulation Variables

An overall risk assessment was performed on the impact of the drug product formulation to the drug product CQAs. Each formulation component that had a high risk of impacting a drug product CQA was further evaluated to reduce the risk. The initial risk assessment of formulation variables is presented in Table 29 and justifications for the risk assignment are presented in Table 30.

**Table 29: Initial Formulation Risk Assessment of Different Variables**

| Drug product CQA | Formulation variables of FDC product | | | | | | |
|---|---|---|---|---|---|---|---|
| | Bempedoic Acid Flow property | Ezetimibe PSD | Bempedoic Acid Granules | Ezetimibe granules Surfactant concentration | Ezetimibe granules Binder concentration | Ezetimibe granules Superdisintegrant concentration | Coating system |
| Assay | Medium | Medium | Medium | Low | Low | Low | Low |
| Content uniformity | Medium | Medium | Medium | Low | Low | Low | Low |
| Dissolution | Low | High | Low | High | Medium | Medium | Low |
| Degradation product | Low | Low | Low | Low | Low | Low | Medium |

**Table 30: Justification of Initial Risk Assessment of Formulation Variables**

| **Drug Substance Attributes** | **Drug product CQA** | **Justification** |
|---|---|---|
| Bempedoic Acid Flow Properties | Assay | Bempedoic acid has poor flow properties which can have impact on assay and CU. The risk is medium. |
| | Content uniformity | |
| | Dissolution | The flowability of the drug substance is unlikely to affect its degradation pathway or solubility. The risk is low. |
| | Degradation product | |
| Ezetimibe PSD | Assay | A small particle size and a wide PSD may adversely impact blend flowability. In extreme cases, poor flowability may impact assay. The risk is medium. |
| | Content uniformity | Particle size distribution has a direct impact on drug substance flowability and ultimately on CU. Improper mixing with excipients may lead to problems in assay and content uniformity. The risk is medium |
| | Dissolution | Drug is BCS class II compound having low solubility. PSD may affect solubility and hence the dissolution. The risk is high. |
| | Degradation product | Particle size is unlikely to impact the degradation products. The risk is low. |
| Bempedoic acid granules | Assay | The poor flow properties of bempedoic acid may result into granules that that can have impact on overall assay and CU. The risk is medium. |
| | Content uniformity | |
| | Dissolution | The flowability of the drug substance is unlikely to affect its degradation pathway or solubility. The risk is low. |
| | Degradation product | |
| Ezetimibe granules | Assay | Surfactant concentration does not affect tablet assay and CU. |
| | Content uniformity | |
| | Dissolution | Ezetimibe is BCS class II compounds having low solubility. For achieving bioequivalence, dissolution of drug substances has to be improved. The risk is high for the ezetimibe fraction of the FDC product. |
| | Degradation product | Proposed surfactant is compatible with active. The risk is low. |
| Ezetimibe granules Binder concentration | Assay | Binder concentration does not affect tablet assay and CU. The risk is low. |
| | Content uniformity | |
| | Dissolution | Binder concentration may affect the release profile. The risk is medium. |
| | Degradation product | Proposed binder is compatible with both the APIs hence it does not affect the degradation of drug product. The risk is low. |
| Ezetimibe granules Superdisintegrant concentration | Assay | Superdisintegrant concentration does not affect tablet assay and CU. The risk is low. |
| | Content uniformity | |
| | Dissolution | Superdisintegrant concentration determines the disintegration time and pattern of tablet. High disintegration time may affect dissolution profile of FDC product. The risk is medium. |
| | Degradation product | Proposed superdisintegrant is compatible hence it does not affect the degradation of drug product. The risk is low. |
| Coating System | Assay | Coating formulation system will not impact assay or content uniformity. The risk low. |
| | Content uniformity | |
| | Dissolution | The selected coating system is removed immediately during dissolution. The risk is low. |
| | Degradation product | The selected coating system contains PEG and PVA which may interact with the API impact stability and increase the impurity. The risk is medium. |

### 6.2.2 Formulation Development Studies

Formulation development focused on evaluation of the high and medium risk formulation variables as identified in the initial risk assessment shown in Table 28. The development was conducted in four stages. The first study optimized the bempedoic acid granulation process. The second study evaluated the impact of the level of povidone in ezetimibe granules on the drug product CQAs by OFAT (one factor at a time). The third study finalized the process for incorporation of sodium lauryl sulfate in the ezetimibe component of the drug product. The fourth study selected an appropriate coating system for the compressed tablet comprising bempedoic acid and ezetimibe.

**Table 31: Ezetimibe Granules Formulation Development Equipment**

| **Sr. No.** | **Process Step** | **Equipment** |
|---|---|---|
| Ezetimibe Granulation (Top spray granulation) | | |
| 1. | Co-sifting of intragranular materials | #40 mesh screen |
| 2. | Dry mixing | Fluid bed processor (Miniglatt) |
| 3. | Binder preparation | Homogenizer |
| | | Magnetic stirrer |
| 4. | Wet granulation | Fluid bed processor (Miniglatt) |
| 5. | Drying | Fluid bed processor (Miniglatt) at 40 - 55° C |
| 6. | Milling and sizing | Sifting the granules through #30 mesh screen |
| 7. | Co-sifting of extragranular material | #30 mesh screen manually |

| Bempedoic Acid Granulation (Wet granulation) | | |
|---|---|---|
| 1. | Co-sifting of intragranular materials | #40 mesh screen |
| | | 5 L Double cone blender |
| 2. | Dry mixing | Rapid mixer granulator 2L |
| 3. | Binder preparation | Magnetic stirrer |
| 4. | Wet granulation | Rapid mixer granulator 2L |
| 5. | Drying | Retsch dryer |
| 6. | Milling and sizing | Sifting the dried granules through #30 mesh screen |
| 7. | Co-sifting of extragranular material | #30 mesh screen manually |

| Compression and Coating | | |
|---|---|---|
| 1. | Blending and lubrication | Double cone blender 5L |
| 2. | Compression | Rimek compression machine 12 station Double rotary compression machine, 28 station (Cadmach) |
| 3. | Coating | Coating machine ideal cure R & D Coater (Gans Coater) |

### Process selection

The development of the fixed dose combination of bempedoic acid and ezetimibe was attempted by fabrication of immediate release tablets in line with reference products. Both APIs were granulated separately using wet granulation. This approach ensured that ezetimibe was treated with SLS to improve solubility and bempedoic acid was treated with colloidal silicon dioxide to avoid sticky behavior. The initial development work was carried out using a monolayer approach.

### Example 2: Formulation Development Study #1: Bempedoic Acid Granules Composition and Process Selection

The goal of Formulation Development Study #1 was to select the bempedoic acid granule composition and process of manufacturing based on a study to reduce the stickiness of the API.

Bempedoic acid exhibited poor flow and sticking during granulation and compression. The sticking of the API was prevented by creating a physical barrier between the API and the contact surface. This was achieved by coating the API with material having high surface area. Colloidal silicon dioxide was selected; it has small particle size and large specific surface area. In the preliminary trials this approach was found to be promising and therefore additional trials were carried out to optimize the concentration of colloidal silicon dioxide and process for this surface treatment.

Optimization trials were initiated using the formulation and process received from Esperion. **Table 32** depicts the formula used for bempedoic acid granulation and **Table 33** describes the trial batches to eliminate sticking behavior.

**Table 32: Formula of Bempedoic Acid Granules**

| Sr. No. | Ingredients | mg/unit |
|---|---|---|
| Intra-granular fraction | | Batch No: 4490-S1-024 |
| 01 | Bempedoic acid (Non-GMP) | 180.00 |
| 02 | Microcrystalline cellulose (Avicel^{®} PH-102) | 51.00 |
| 03 | Lactose monohydrate (Pharmatose^{®} 200M) | 30.00 |
| 04 | Sodium starch glycolate (Primojel^{®}) | 14.00 |
| 05 | Hydroxypropyl cellulose (HPC-L) | 12.00 |
| 06 | Purified water | q.s. |
| Total weight of granules | | 287.00 |

| Extra-granular fraction | | Batch No: 4490-S1-024 |
|---|---|---|
| 07 | Magnesium stearate | 4.5 |
| 08 | Sodium starch glycolate (Primojel^{®}) | 7.0 |
| 09 | Colloidal silicon dioxide (Aerosil^{®} 200P) | 1.5 |
| Weight of tablet (bempedoic acid fraction) | | 300.00 |

**Table 33: Trial Batches to Eliminate Sticking of Bempedoic Acid Tablets During Compression**

| **Batch No./ Parameter** | **4490-S1-024 (Esperion process)** | **4490-S1-030** | **4490-S1-042** | **4759-S1-096** |
|---|---|---|---|---|
| Concentration of Aerosil^{®} 200P (mg/tablet) | 1.5 | 6.5 | 4.0 | 4.0 |
| Duration of treatment (mins) | 10 | 30 | 30 | 45 |
| Manufacturing process used | Wet granulation | Wet granulation | Wet granulation | Wet granulation |

Sticking behavior could be significantly reduced by treating with colloidal silicon dioxide at increasing concentrations prior to granulation. Furthermore, maintaining the level of colloidal silicon dioxide and increasing the duration of the surface treatment from 30 min to 45 mins, resulted in satisfactory processing parameters without any sticking during granulation or compression processes. The final composition of bempedoic acid granules is listed in **Table 34.**

**Table 34: Final Composition of Bempedoic Acid Granules (Batch No: 4759-S1-093)**

| Sr. No. | Ingredients | mg/unit |
|---|---|---|
| 1 | Bempedoic acid | 180.00 |
| 2 | Colloidal silicon dioxide (Aerosil^{®} 200P) | 3.50 |
| 3 | Microcrystalline cellulose (Avicel^{®} PH-102) | 09.60 |
| 4 | Hydroxypropyl cellulose (HPC-L) | 12.00 |
| | Total weight of bempedoic acid granules | 205.10 |

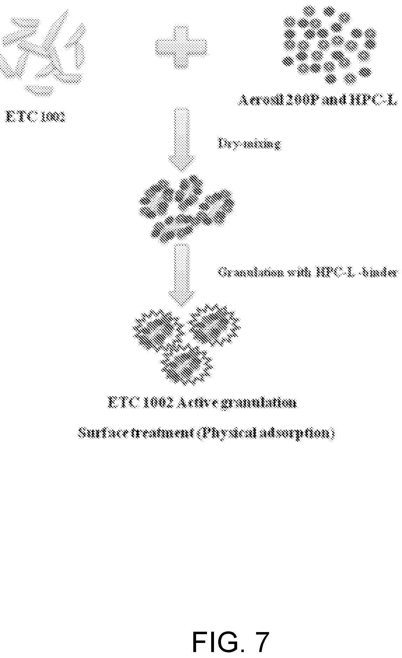

Table 35 provides the process parameters selected for further development work. A depiction of the treatment process is shown in **Figure 7****.**

**Table 35: Process Parameters for Bempedoic Acid Granulation**

| Batch No: 4759-S1-093 | | Batch No: 2000 tablets |
|---|---|---|
| Sr. No. | Process Step | Parameter |
| 1. | Dry mixing time | 45 minutes |
| 2. | Binder addition time | 1.5 minutes |
| 3. | Kneading time | 2.5 minutes |
| 4. | Wet sizing | Through #18 mesh |
| 5. | Drying temperature | 45°C |
| 6. | LOD of dried granules | NMT 1% at 50°C |
| 7. | Sifting of dried granules | #30 mesh sieve |

### Example 3: Formulation Development Study #2: Optimization of Ezetimibe Granules:

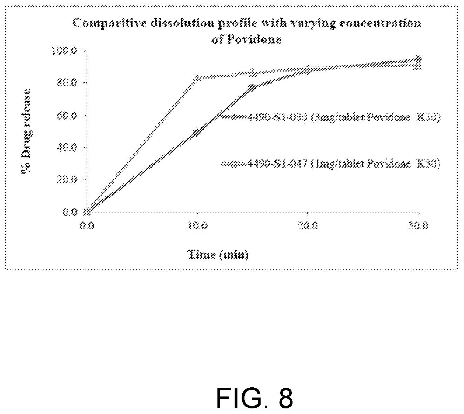

The goal of Formulation Development Study #2 was to optimize the concentration of binder used for the granulation of ezetimibe. Wet granulation was used for the preparation of ezetimibe granules. Binder solution, povidone with SLS, and ezetimibe were used for the granulation of dry-mix powder blend. One trial with a higher concentration of povidone (3 mg/tablet) and another with a lower concentration (1 mg/tablet) were performed to observe the impact of binder concentration on dissolution. Ezetimibe granules were mixed with Bempedoic acid granules separately and compressed into a monolayer tablet for both trials. A summary of the formulations is provided in **Table 36.** The dissolution profiles were studied in QC media (with 0.45% SLS) and are shown in **Table 37** and **Figure 8****.**

**Table 36: Formula of Ezetimibe Granules with Varying Concentration of Binder**

| Ingredients | mg/unit | |
|---|---|---|
| Ezetimibe granulation | 4490-S1-030 | 4490-S1-047 |
| Ezetimibe | 10.00 | 10.00 |
| Lactose monohydrate (Pharmatose^{®} 200M) | 50.00 | 50.00 |
| Microcrystalline cellulose (Avicel^{®} PH-102) | 19.00 | 21.00 |
| Sodium starch glycolate (Primojel^{®}) | 6.00 | 6.00 |

| Binder solution | | |
|---|---|---|
| Povidone (Kollidon^{®} 30) | 3.00 | 1.00 |
| Sodium laurvl sulfate (Kolliphor^{®} SLS Fine) | 2.00 | 2.00 |
| Purified water | q.s. | q.s. |
| Total weight of granules | 90.00 | 90.00 |

**Table 37: Dissolution of Ezetimibe with Varying Concentration of Binder**

| Time (mins) | 4490-S1-030 (3mg/tablet povidone K30) % Drug Release | 4490-S1-047 (1mg/tablet povidone K30) % Drug Release |
|---|---|---|
| 0.0 | 0.0 | 0.0 |
| 10.0 | 49.4 | 82.7 |
| 15.0 | 77.0 | 86.0 |
| 20.0 | 87.5 | 88.8 |
| 30.0 | 94.2 | 90.9 |

Tablets with higher binder concentration initially showed slower release than the tablets with lower binder concentration. Higher binder concentration may retard the release from the granules initially after disintegration of tablets. Therefore 1 mg/tablet binder concentration was selected for ezetimibe granulation.

### Example 4: Formulation Development Study # 3: Ezetimibe Granules - Optimization of SLS Incorporation Process:

The sodium lauryl sulfate concentration was estimated from the reference product, Zetia, using titration. Zetia was measured to contain 1.8 mg (~ 2.0 mg) SLS per tablet. The same concentration of surfactant was considered for the ezetimibe granulation process to achieve a matching dissolution profile.

Initially, ezetimibe granules were prepared by wet granulation. The granulation process included dry-mixing ezetimibe with diluents, MCC and lactose, and superdisintegrant, SSG, followed by granulation with a binder solution containing povidone K30 and sodium lauryl sulfate as surfactant in purified water. However, the dissolution profile obtained was slower than the Zetia dissolution profile. Therefore, a series of process modification trials were carried out.

### Co-Sifting of Ezetimibe with Hydrophilic Excipients (Batch No: 4759-S1-064)

Ezetimibe was co-sifted with hydrophilic excipients, lactose monohydrate (Pharmatose^{®} 200M), Polyvinyl pyrrolidone (Kollidon^{®} 30), through #50 mesh to reduce hydrophobicity. The blend was then granulated with a binder solution containing SLS. Ezetimibe granules were then blended with extra granular excipients and compressed into tablets. This trial did not yield the desired improvement in dissolution.
Homogenization of binder solution with ezetimibe followed by RMG granulation (4759-S1-065)
When granulated with SLS, the surfactant was distributed over the entire mass, including the excipient. As a result, the desired improvement in dissolution was not achieved. To provide adequate contact between ezetimibe and SLS, ezetimibe was added to the binder solution and then homogenized for 30 minutes to achieve uniform dispersion. This dispersion was then granulated with the excipient blend. The granules were then blended with extra granular excipients and compressed into a tablet. Though the dissolution profile improved, it was not equivalent to the dissolution profile of Zetia.
Homogenization of binder solution with ezetimibe followed by top spray granulation (Batch No: 4759-S1-094)

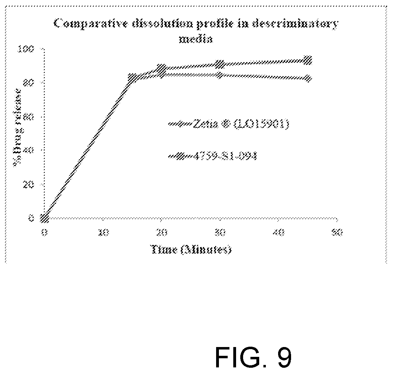

To further improvement the dissolution profile, the homogenized binder solution was incorporated with top spray granulation using a fluid bed processer (FBP) instead of rapid mixer granulator. The granules were subsequently blended with extra granular excipients and compressed into tablets. The dissolution in discriminatory dissolution media obtained is shown in Table 38 and Figure 9.

**Table 38: Dissolution Profile in Discriminatory Dissolution Medium**

| Time(min) | % Drug release Zetia (10 mg) (LO15901) | 4759-S1-094 |
|---|---|---|
| 0 | 0 | 0 |
| 15 | 81.8 | 82.9 |
| 20 | 84.8 | 88.4 |
| 30 | 84.6 | 90.9 |
| 45 | 82.5 | 93.1 |

Process Parameters for Ezetimibe Granulation (Top Spray Method): The process parameters listed in Table 39 were employed for ezetimibe granulation:

**Table 39: Process Parameters for Ezetimibe Granulation**

| **Batch No: 4759-S1-094** | | **Batch** | |
|---|---|---|---|
| **Sr. No.** | **Process parameters** | | **Details** |
| **1.** | **Binder solution preparation** | | |
| | Homogenization time | | 30 minutes |
| **2.** | **Granulation process** | | |
| | Product bed temperature | | 30-45° C |
| **3.** | **Drying** | | |
| | Product bed temperature | | 45-60° C |
| | LOD of dried granules | | 1.73% (NMT 2% at 105° |
| | Sifting of dried granules | | #30 mesh sieve |

| | | | |
|---|---|---|---|
| 2000 tablets | | | |

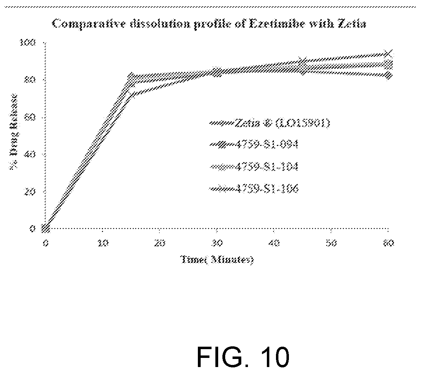

Two reproducibility batches (Batch no: 4759-S1-104 and 4759-S1-106) were manufactured using the same set of parameters. Dissolution profiles obtained from the reproducibility trials are shown in Table 40 and comparative dissolution profile is shown in **Figure 10****.**

**Table 40: Dissolution Profile for Process Reproducibility Trials**

| **Time (min)** | **Zetia (10 mg) (LO15901)** | **4759-S1-094** | **4759-S1-104** | **4759-S1-106** |
|---|---|---|---|---|
| 0 | 0 | 0 | 0 | 0 |
| 15 | 81.8 | 78.4 | 80.4 | 71.9 |
| 30 | 84.8 | 83.7 | 85.0 | 84.6 |
| 45 | 84.6 | 86.0 | 87.4 | 90.0 |
| 60 | 82.5 | 88.1 | 88.9 | 93.8 |

The reproduced batches showed similar release profiles to the previous batch and reference product. The process can be considered reproducible at laboratory scale. Formulation Development Study # 4: Coating System Selection

During drug-excipient compatibility studies, Opadry white 85F18422 was found to be incompatible with ezetimibe. This observation was confirmed during stability studies of tablets coated with Opadry white. Assay and impurity profile are given in Table 41.

**Table 41: Assay and Impurity Profile at Stability Condition**

| Batch No | Condition | Specified Impurities (EZT) | | Single max unspecified impurities | Total impurities | Assay (%) | |
|---|---|---|---|---|---|---|---|
| | | Cyclic Ether | EZT 21 (Keto impurity) | | | EZT | Bempedoic acid |
| 4490-S1-047 | Initial | 0.02 | 0.05 | BLOQ | 0.07 | 100.0 | 100.1 |
| | 1M 40°C / 75% RH | 0.11 | 0.04 | BLOQ | 0.15 | 96.6 | 99.1 |
| ND-Not detected, BLOQ- Below limit of quantitation | | | | | | | |

Opadry white (85F18422) is a polyvinyl alcohol (PVA) based coating system with polyethylene glycol (PEG) employed as a plasticizer. The increase in cyclic ether impurity was attributed to either of these materials. Therefore, the following Opadry coating systems were studied for compatibility with ezetimibe. To validate the results, the compressed tablets were coated with these Opadry systems and subjected to a stress study.
PVA & PEG containing Opadry system (Opadry White 88A180040)
PVA based PEG -free Opadry white (Opadry White AMB II 88A180040)
PVA and PEG -free HPMC based Opadry white (Opadry White 03K58821).

The impurity profile post exposure for one week at 600C in an open container was employed as response for comparative evaluation. The results are provided in **Table 42.**

**Table 42: Impurity Profile of Bempedoic Acid and Ezetimibe with different Opadry systems**

| Name of Opadry | Opadry White 85F18422 | | Opadry AMB II 88A180040 White | | | Opadry White 03K58821 | | |
|---|---|---|---|---|---|---|---|---|
| | PVA and PEG based Opadry | | PVA (w/o PEG) based Opadry) | | | HPMC based Opadry | | |
| **Sample** | **Condition** | **Impurity profile** | | | | | | |
| | | **EZT cyclic ether** | | **RRS isomer** | **EZT 21 (Keto impurity)** | | **SMUI** | **Total impurities** |
| Ezetimibe + Bempedoic acid + Opadry white (85F 18422) | Initial | ND | | ND | 0.03 | | ND | 0.03 |
| | 2W 60° C | 9.05 | | ND | 0.13 | | 1.38 | 11.06 |
| | closed | | | | | | | |
| | 4W 40° C / 75% RH | 6.27 | | ND | 0.08 | | 0.11 | 0.29 |
| | Closed | | | | | | | |
| Ezetimibe + Bempedoic acid + Opadry White AMB II 88A180040 | Initial | 0.01 | | ND | 0.03 | | 0.02 | 0.07 |
| | 2W 60° C | 0.02 | | ND | 0.04 | | 0.02 | 0.14 |
| | closed | | | | | | | |
| | 4W 40° C / 75% RH | 0.03 | | ND | 0.04 | | 0.02 | 0.11 |
| | Closed | | | | | | | |
| Ezetimibe + Bempedoic acid + Opadry White AMB II 03K58821 | Initial | ND | | ND | 0.04 | | 0.02 | 0.08 |
| | 2W 60° C | ND | | ND | 0.04 | | 0.02 | 0.08 |
| | closed | | | | | | | |
| | 4W 40° C / 75% RH | ND | | ND | 0.05 | | 0.03 | 0.10 |
| | Closed | | | | | | | |

### Accelerated Stability Data:

Stress stability data of compressed tablets coated with different Opadry system at 60°C for 1 week is given in Table 43.

**Table 43: Stress Stability Data of Tablets Coated with Different Opadry systems at 60° C**

| | | | |
|---|---|---|---|
| Impurity | 4490-S1-030 (Opadry white 88A180040) | 4490-S1-052A (Coated with AMB II 88A180040 White) | 4490-S1-052B (Opadry White 03K58821) |
| EZT (Cyclic ether impurity) | 0.61 | 0.02 | 0.01 |
| EZT (Keto impurity) | 0.07 | 0.04 | 0.04 |
| Single max unspecified impurity | 0.06 | BLOQ | BLOQ |
| Total impurities | 0.74 | 0.06 | 0.04 |

The study confirmed previous observations of drug instability with Opadry White 88A180040. The alternate Opadry systems, Opadry White AMB II 88A180040 and Opadry White 03K58821, were found to be compatible with the APIs as reflected by the level of ezetimibe cyclic ether impurity. These results were confirmed during the stress stability studies on the coated tablet formulation. Among the two Opadry systems; Opadry White was AMB II 88A180040 selected as it did not contain the polyethylene glycol and exhibited relatively superior processibility.

### 6.2.3 Updated Risk Assessment of Drug Substance Attributes 6.2.3

Table 44 and Table 45 summarize the updated risk assessment of the drug substance attributes of bempedoic acid and ezetimibe.

**Table 44: Bempedoic Acid Updated Risk Assessment & Justification of the Drug Substance Attributes**

| **Drug substance attributes** | **Drug Products CQAs** | **Updated risk** | **Justification** |
|---|---|---|---|
| **Particle Size Distribution** | Dissolution | Low* | The impact of change in particle size of the drug substance upon the dissolution profile is known (dissolution study with coarser and fine grade API). Control of the drug substance particle size is critical. The risk was reduced from medium to low. |
| **Solubility** | Dissolution | Low* | Bempedoic acid shows pH dependent dissolution profile. Solubility increases at higher pH. In QC media (phosphate buffer pH 6.8) formulation shows comparable release profile with reference product. The risk was reduced from medium to low |
| **Flow Properties** | Assay | Low* | Granulation process improves the flow of bempedoic acid which reduces the risk related to the assay and content uniformity during processing. The risk was reduced from medium to low |
| | Content | | |
| **Total impurity** | Degradation product | Low* | Controlled impurity level in API & also no unspecified impurity generated in the formulation. The risk was reduced from medium to low. |

| | | | |
|---|---|---|---|
| * For validation with multiple API batches | | | |

**Table 45: Ezetimibe Updated Risk Assessment & Justification of the Drug Substance Attributes**

| **Drug substance attributes** | **Drug Products CQAs** | **Updated risk** | **Justification** |
|---|---|---|---|
| **Particle Size Distribution (PSD)** | Assay | Low* | Using top spray wet granulation gave size enlargement and improved flow significantly with satisfactory assay & CU. The risk was reduced from medium to low. |
| | CU | | |
| | Dissolution | | Micronized API was selected from TEVA. Homogenization was adapted to improve dissolution of ezetimibe. The risk was reduced from high to low. |
| **Solubility** | Dissolution | Low* | 2.0 mg SLS per tablet was used with homogenization prior to top spray granulation process. The risk was reduced from medium to low. |
| **Chemical Stability** | Degradation product | Low* | Impurity levels are controlled in API & taken necessary precaution during formulation to reduce the level of degradation. The risk was reduced from medium to low. |
| **Flow Properties** | Assay | Low* | Wet granulation approach improves the flow with satisfactory assay and CU. The risk was reduced from medium to low. |
| | CU | | |
| **Process impurity** | Degradation product | Low* | Process impurity of drug substance was controlled in API and selected compatible excipients in the formulation to reduce the degradation. Also monitored during stability. The risk is reduced from medium to low. |

| | | | |
|---|---|---|---|
| * For validation with multiple API batches | | | |

### 6.2.4 Updated Risk Assessment of the Formulation Components:

Acceptable ranges for the high risk formulation variables have been established and were included in the control strategy. Based on the results of the formulation development studies, the risk assessment of the formulation variables was updated in Table 46.

**Table 46: Updated Formulation Risk Assessment & Justification**

| **Drug substance attributes** | **Drug Products CQAs** | **Updated risk** | **Justification** |
|---|---|---|---|
| **Ezetimibe granules (Surfactant concentration)** | Dissolution | Low* | From the reverse engineering of Zetia, the selected concentration of SLS, 2.0mg/tablets, showed satisfactory dissolution. The risk was reduced from high to low. |
| **Ezetimibe granules (Binder concentration)** | Dissolution | Low* | From the trials with varying concentration of binder, the selected concentration of povidone, 1.0mg/tablets, shows satisfactory dissolution. The risk was reduced from medium to low. |
| **Coating system** | Degradation product | Low* | From the compatibility study, the selected PEG free Opadry system Opadry AMB II 88A180040 White shows significantly lower degradation as compared to Opadry with PEG. |
| | | | The risk was reduced from medium to low. |

| | | | |
|---|---|---|---|
| * For validation with multiple API batches | | | |

### 6.3 Manufacturing Process Development

Two approaches were used for manufacturing of FDC tablets: a monolayer and a bilayer. Batches produced where put on a stability study.

The monolayer process included manufacturing ezetimibe and bempedoic acid granules separately, blending them together, and compressing to a single layer tablet. The

### tablet was then coated.

The bilayer approach involved blending bempedoic acid granules with extra-granular excipients and blending ezetimibe granules with extragranular excipients. The two lubricated blends were s compressed into a two layered FDC tablet, one layer comprising bempedoic acid and the other comprising ezetimibe. The bilayer FDC tablets were then coated.

### 6.3.1 FDC-Monolayer Tablet:

Table 47 provides the composition of the monolayer tablet.

**Table 47: Composition Details of Optimized Formulation for FDC-Monolayer Product**

| **Ingredients** | **mg/unit** |
|---|---|
| **Ezetimibe granulation (Batch no: 4759-S1-094 (Batch size: 2000 units)** | |
| Ezetimibe | 10.00 |
| Lactose monohydrate (Pharmatose^{®} 200M) | 50.00 |
| Microcrystalline cellulose (Avicel^{®} PH-102) | 11.00 |
| Sodium starch glycolate (Primojel^{®}) | 6.00 |
| Kollidone^{®} 30 (povidone K30) | 1.00 |
| Sodium lauryl sulfate (Kolliphor^{®} SLS Fine) | 2.00 |
| Purified water | q.s. |
| **Total weight of granules** | **80.00** |

| **Bempedoic acid granulation (Batch no: 4490-S1-093) (Batch size: 2000 units)** | |
|---|---|
| Bempedoic acid | 180.00 |
| Colloidal silicon dioxide (Aerosil^{®} 200P) | 3.50 |

| **Ingredients** | **mg/unit** |
|---|---|
| Microcrystalline cellulose (Avicel^{®} PH-102) | 9.60 |
| Hydroxypropyl cellulose (HPC-L) | 12.00 |
| **Total weight of bempedoic acid granules** | **205.10** |

| **Extra granular excipients (Batch no: 4490-S1-096) (Batch size: 1000 units)** | |
|---|---|
| Ezetimibe granules | 80.00 |
| Bempedoic acid granules | 205.1 |
| Sodium starch glycolate (Primojel^{®}) | 21.00 |
| Lactose monohydrate (Pharmatose^{®} 200M) | 25.00 |
| Microcrystalline cellulose (Avicel^{®} PH-102) | 40.00 |
| Colloidal silicon dioxide (Aerosil^{®} 200P) | 0.50 |
| Magnesium stearate | 4.00 |
| **Total weight of core tablets** | **375.60** |
| Opadry ABMII white 88A180040 | 9.40 |
| **Weight of coated tablet** | **385.00** |

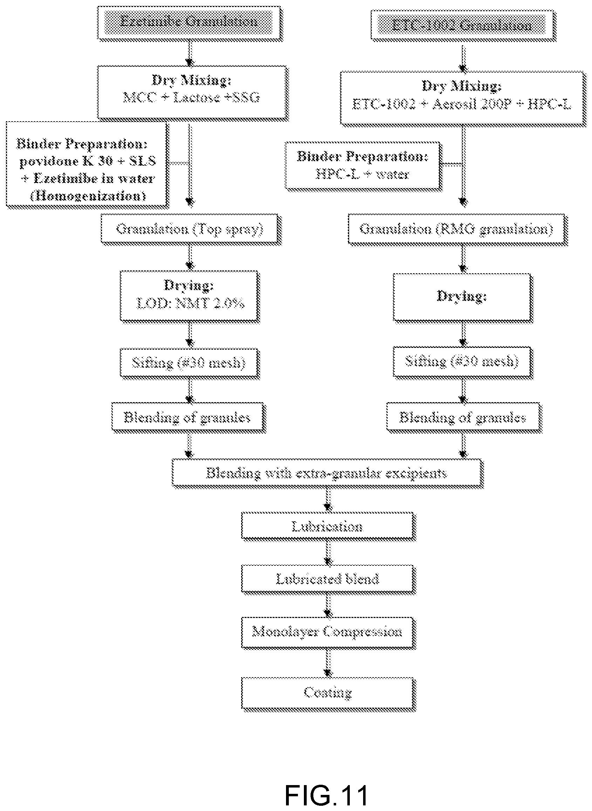

The FDC-monolayer tablet manufacturing process is shown in Figure 11. 6.3.2 FDC-Bilayer Tablet

The composition of final formulation of bilayer tablet is presented in Table 48.

**Table 48: Bilayer FDC Product Composition**

| **Ingredients** | **mg/unit** |
|---|---|
| **Ezetimibe granulation (Batch no: 4759-S1-104 (Batch size: 2000 units)** | |
| Ezetimibe | 10.00 |
| Lactose monohydrate (Pharmatose^{®} 200M) | 50.00 |
| Microcrystalline cellulose (Avicel^{®} PH-102) | 11.00 |
| Sodium starch glycolate (Primojel^{®}) | 6.00 |
| Kollidone^{®} 30 (povidone K30) | 1.00 |
| Sodium lauryl sulfate (Kolliphor^{®} SLS) | 2.00 |
| Microcrystalline cellulose (Avicel^{®} PH-102) | 9.00 |
| Magnesium stearate | 1.00 |
| Purified water | q.s. |
| **Total weight of lubricated blend** | **90.00** |

| **Bempedoic acid granulation (Batch no: 4759-S1-101) (Batch size: 2000 units)** | |
|---|---|
| Bempedoic acid | 180.00 |

| **Ingredients** | **mg/unit** |
|---|---|
| Colloidal silicon dioxide (Aerosil^{®} 200P) | 3.50 |
| Microcrystalline cellulose (Avicel^{®} PH-102) | 09.60 |
| Hydroxypropyl cellulose (HPC-L) | 12.00 |
| Sodium starch glycolate (Primojel^{®}) | 21.00 |
| Lactose monohydrate (Pharmatose^{®} 200M) | 25.00 |
| Microcrystalline cellulose (Avicel^{®} PH-102) | 31.00 |
| Colloidal silicon dioxide (Aerosil^{®} 200P) | 0.50 |
| Magnesium stearate | 3.00 |
| **Total weight of lubricated blend** | **285.6** |

| **Component of Bilayer compression: (Batch no: 4759-S1-111) (Batch size: 1000 units)** | |
|---|---|
| Ezetimibe lubricated blend | 90.00 |
| Bempedoic acid lubricated blend | 285.6 |
| **Total weight of core tablets** | **375.60** |
| Opadry ABMII white 88A180040 | 9.40 |
| **Weight of coated tablet** | **385.00** |

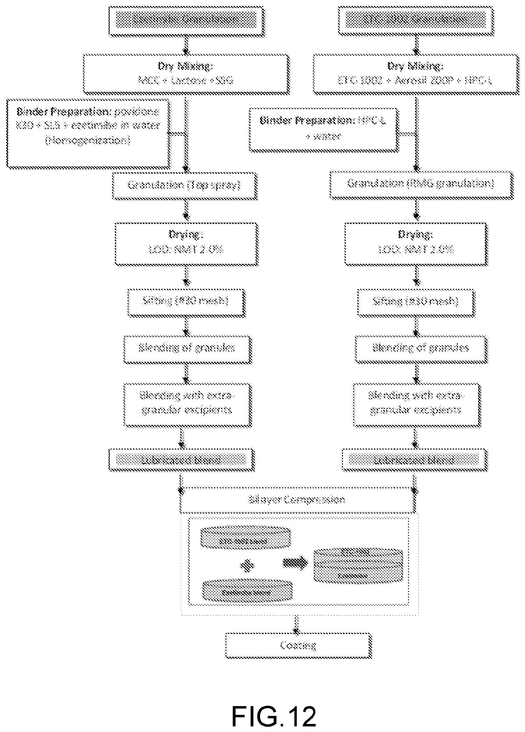

The FDC-bilayer tablet manufacturing process is depicted in Figure 12.

Physical Parameters of Compressed Tablets. Table 49 includes the compression parameters for both tablet variants:

**Table 49: Compression Parameters of FDC Tablets**

| **Batch Number** | **FDC-Monolayer tablet 4490-S1-096** | **FDC-Bilayer tablet 4759-S1-111** |
|---|---|---|
| Individual tablet weight (mg) | 368 - 380 | 373 - 380 |
| Hardness (N) | 80 - 90 | 80 - 110 |
| Thickness (mm) | 4.50 - 4.55 | 4.31 - 4.38 |
| Friability (%) | 0.20 | 0.18 |
| Disintegration time (min) | 2 to 4 minutes | 3 to 5 minutes |

Observations: Physical parameters of lubricated blend and compression tablets were found to be satisfactory for both variants. Coating parameters were found to be satisfactory.

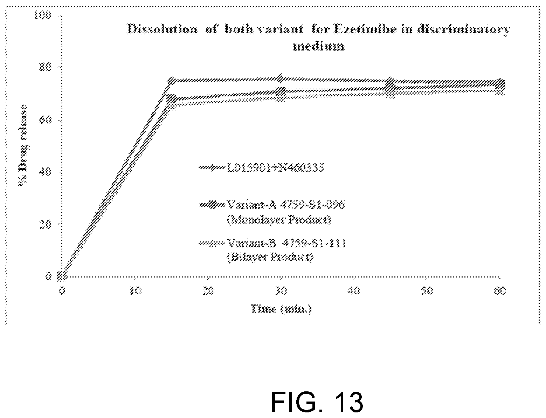

Dissolution of FDC formulations in discriminatory dissolution media: Dissolution profile for ezetimibe in discriminatory dissolution media: Comparative dissolution profile of ezetimibe from both variants of FDC product vs. Zetia (10 mg) + Bempedoic acid (180 mg) is shown in Table 50 and Figure 13.Table 50: Ezetimibe release from monolayer and bilayer FDC in discriminatory media

**Table 50: Ezetimibe release from monolayer and bilayer FDC in discriminatory media**

| **Dissolution comparison with Zetia (10 mg)** | | | |
|---|---|---|---|
| **Dissolution condition:** 0.1% SLS in 0.05M acetate buffer, pH 4.5 USP Apparatus -II, 50 rpm, 900mL | | | |
| **Product** | **Zetia (10 mg) + Bempedoic acid (180 mg)** (Reference Product) | **FDC Product** | |
| **Batch/Lot No.** | L015901+N460335 | FDC-Monolayer Tablet 4759-S1-096 | FDC-Bilayer Tablet 4759-S1-111 |
| 15 | 74.7 | 67.5 | 65.5 |
| 30 | 75.6 | 70.5 | 68.4 |
| 45 | 74.6 | 71.7 | 70.0 |
| 60 | 74.3 | 73.1 | 71.2 |

Conclusion: The dissolutions of both the monolayer and bilayer FDC tablets were found to be comparable to the dissolution Zetia (10 mg) (ezetimibe) + bempedoic acid (180 mg) (reference product).

### Dissolution Profile for Bempedoic Acid in Discriminatory Dissolution Medium:

The QC release media, phosphate buffer, pH 6.8, exhibited dose dumping (almost above 90% in 15 minutes). Based on the optimization of surfactant concentration (0.1% to 0.45%) and dissolution volume (500 mL to 1000 mL), 0.45% SLS in acetate buffer, pH 4.5, 1000 mL, 50 rpm, paddle (USP App-II) gave gradual release profile as compared to QC media.

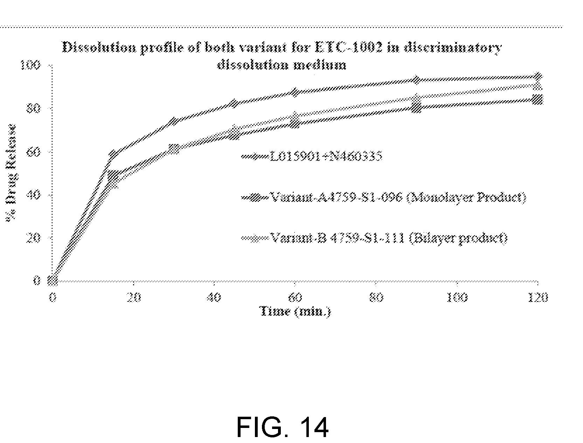

Table 51 and Figure 14 show comparative dissolution profiles of Bempedoic acid from both variates of the FDC product vs Zetia (10 mg) + bempedoic acid (180 mg) (reference product).

**Table 51: Dissolution Profile for Bempedoic Acid in Discriminatory Dissolution Media**

| **Dissolution comparison for BEMPEDOIC ACID** | | | |
|---|---|---|---|
| **Dissolution condition: 0.45% SLS in acetate buffer, pH 4.5, 1000 mL, 50 rpm, paddle (USP App-II)** | | | |
| **Product** | **Zetia (10 mg) + Bempedoic acid (180 mg)** (Reference Product) | **FDC Product** | |
| **Batch No.** | L015901+N460335 | Monolayer (4759-S1-096) | Bilayer (4759-S1-111) |
| **Time (min.)** | **% Drug Release (Bempedoic acid)** | | |
| 15 | 58.8 | 48.9 | 45.4 |
| 30 | 73.9 | 61.2 | 61.1 |
| 45 | 82.2 | 67.8 | 70.4 |
| 60 | 87.5 | 72.9 | 76.5 |
| 90 | 93.1 | 80.4 | 85.1 |
| 120 | 94.7 | 84.2 | 90.9 |

The % drug release of BEMPEDOIC ACID from both variants of the FDC exhibit release profiles comparable to the reference product.

Dissolution Profile of FDC Prototype Product (Ezetimibe Component) in QC Dissolution medium:

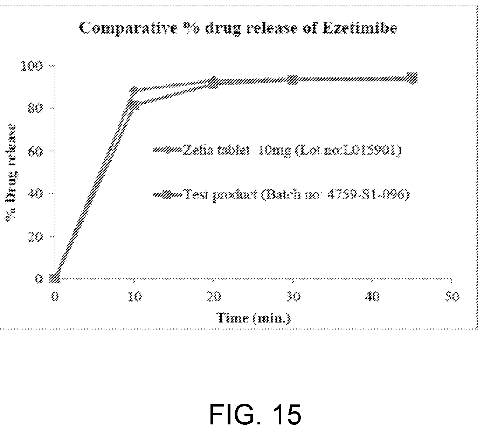

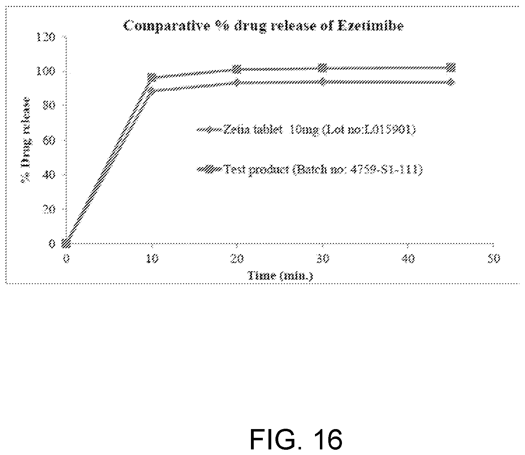

Dissolution Profile of FDC test product (ezetimibe component) in QC media is given in Table 52, Table 53, Figure 15, and Figure 16.

**Table 52: Dissolution Profile of Ezetimibe Component from FDC-Monolayer Tablet (Batch No: 4759-S1-096)**

| **Dissolution condition** | | |
|---|---|---|
| 0.45% SLS in acetate buffer, pH 4.5, USP Apparatus -II, 50 rpm, 500 mL | | |
| **Product** | Zetia (10 mg) | Prototype Product |
| **Batch No.** | L015901 | 4759-S1-096 |
| Time (min.) | % Drug Release (ezetimibe) | |
| 10 | 88.4 | 81.5 |
| 20 | 93.2 | 91.5 |
| 30 | 93.7 | 93.5 |
| 45 | 93.5 | 94.4 |

**Table 53: Dissolution Profile of Ezetimibe Component of FDC-Bilayer Tablet (Batch no: 4759-S1-111)**

| **Dissolution condition** | | |
|---|---|---|
| 0.45% SLS in 0.05M acetate buffer, pH 4.5, USP Apparatus -II, 50 rpm, 500 mL | | |
| **Product** | Zetia (10 mg) | Prototype Product |
| **Batch No.** | L015901 | 4759-S1-111 |
| Time (min.) | % Drug Release (Ezetimibe) | |
| 10 | 88.4 | 96.1 |
| 20 | 93.2 | 101.0 |
| 30 | 93.7 | 101.7 |
| 45 | 93.5 | 102.1 |

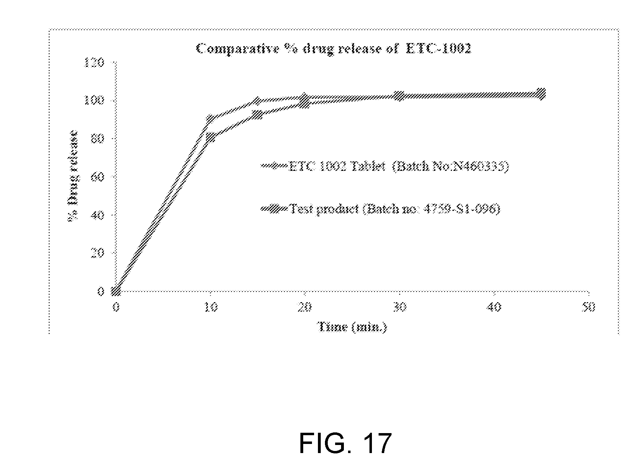

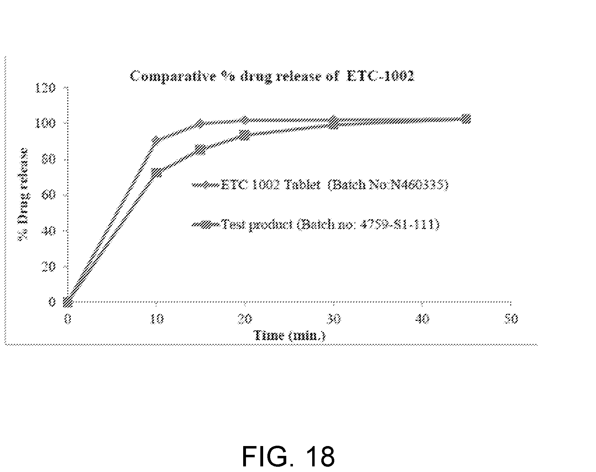

Dissolution of both the variants (monolayer and bilayer) showed similar dissolution to the reference product, Zetia (10 mg) in QC media. Dissolution profile of FDC test product (bempedoic acid component) in QC media: Dissolution profile of FDC test product (bempedoic acid component) is given in Table 54, Table 55, Figure 17, and Figure 18.

**Table 54: Dissolution Profile of Bempedoic Acid Component of FDC-Monolayer Tablet (Batch No: 4759-S1-096)**

| **Dissolution condition** | | |
|---|---|---|
| 50 mM phosphate buffer, pH 6.8, USP Apparatus -II, 50 rpm, 900 mL | | |
| **Product** | Bempedoic acid (180 mg) Reference Product | FDC - Monolayer Test product |
| **Batch No.** | N460335 | 4759-S1-096 |
| Time (min.) | % Drug Release (Bempedoic acid) | |
| 10 | 90.2 | 80.5 |
| 15 | 99.6 | 92.5 |
| 20 | 101.6 | 98.2 |
| 30 | 101.8 | 102.4 |
| 45 | 102.2 | 103.7 |

**Table 55: Dissolution Profile of Bempedoic Acid Component of FDC-Bilayer Tablet (Batch no: 4759-S1-111)**

| **Dissolution condition** | | |
|---|---|---|
| 50 mM phosphate buffer, pH 6.8, USP Apparatus -II, 50 rpm, 900 mL | | |
| **Product** | Bempedoic acid (180 mg) Reference Product | BFDC - Bilayer Test product |
| **Batch No.** | N460335 | 4759-S1-111 |
| Time (min.) | % Drug Release (Bempedoic acid) | |
| 10 | 90.2 | 72.5 |
| 15 | 99.6 | 85.1 |
| 20 | 101.6 | 93.2 |
| 30 | 101.8 | 99.1 |
| 45 | 102.2 | 102.4 |

Dissolution of bempedoic acid from both variants showed similar dissolution profiles to the reference product in QC medium.

### 6.4 Stability Study

For stability loading, 30 tablets were packed in 30 cc HDPE bottles with 1 g polyester coil and 1g sorbit canister. The bottles were sealed with 28 mm CRC caps.

### 6.4.1 Stability data of FDC-monolayer variant:

Stability data for the monolayer tablets is given in Table 56.

**Table 56: Stability Data of FDC-Monolayer Tablets (Batch no: 4759-S1-096)**

| **Sr. No.** | **Test** | | **Initial** | **Condition: 40°/75% RH** | |
|---|---|---|---|---|---|
| | | | | **1M** | **2M** |
| 01 | Physical appearance | | White colored, oval shaped film coated tablet debossed "000" on one side and "ABC" on other side | Complies | |
| 02 | Disintegration time (min) | | 2-3 minutes | 3-4 minutes | 2-3 minutes |
| 03 | Hardness (N) | | 98-100 | 100-123 | 103-112 |
| 04 | Water by KF (%w/v) | | 2.96 | 2.65 | 2.45 |
| 03 | Assay (%) | Ezetimibe | 103.2 | 104.0 | 104.9 |
| | | Bempedoic acid | 103.9 | 100.0 | 100.0 |

| | Impurity | Ezetimibe | | | |
|---|---|---|---|---|---|
| 04 | | Cyclic Ether | ND | BLOQ | BLOQ |
| | | EZT 21(Keto impurity) | BLOQ | BLOQ | 0.09 |
| | | Single max unspecified impurities | BLOQ | BLOQ | BLOQ |
| | | Total impurities | BLOQ | BLOQ | 0.09 |

| | | Bempedoic acid | | | |
|---|---|---|---|---|---|
| | | Unspecified impurity | ND | ND | 0.06 |
| 05 | Dissolution | Time (min) | | | |
| For ezetimibe: 0.45% SLS in 0.05 M acetate buffer, pH 4.5, USP Apparatus -II, 50 rpm, 500 ml | | 10 | 81.5 | 85.8 | 71.1 |
| | | 20 | 91.5 | 94.0 | 95.4 |
| | | 30 | 93.5 | 95.8 | 100.6 |
| | | 45 | 94.4 | 96.8 | 102.1 |
| For bempedoic acid: 50mM phosphate buffer, pH 6.8, USP | | Time (min) | | | |
| | | 10 | 84.9 | 75.3 | 77.0 |
| | | 20 | 100.8 | 93.4 | 95.2 |

| **Sr. No.** | **Test** | | **Initial** | **Condition: 40°/75% RH** | |
|---|---|---|---|---|---|
| | | | | **1M** | **2M** |
| 01 | Physical appearance | | White colored, oval shaped film coated tablet debossed "000" on one side and "ABC" on other side | Complies | |
| 02 | Disintegration time (min) | | 2-3 minutes | 3-4 minutes | 2-3 minutes |
| 03 | Hardness (N) | | 98-100 | 100-123 | 103-112 |
| 04 | Water by KF (%w/v) | | 2.96 | 2.65 | 2.45 |
| 03 | Assay (%) | Ezetimibe | 103.2 | 104.0 | 104.9 |
| | | Bempedoic acid | 103.9 | 100.0 | 100.0 |

| | Impurity | Ezetimibe | | | |
|---|---|---|---|---|---|
| 04 | | Cyclic Ether | ND | BLOQ | BLOQ |
| | | EZT 21(Keto impurity) | BLOQ | BLOQ | 0.09 |
| | | Single max unspecified impurities | BLOQ | BLOQ | BLOQ |
| | | Total impurities | BLOQ | BLOQ | 0.09 |

| | | Bempedoic acid | | | |
|---|---|---|---|---|---|
| | | Unspecified impurity | ND | ND | 0.06 |
| Apparatus -II, 50 rpm, 900 ml | | 30 | 104.8 | 99.3 | 100.5 |
| | | 45 | 106.5 | 102.2 | 104.1 |

| | | | | | |
|---|---|---|---|---|---|
| BLOQ- Below limit of quantification <0.05%, ND- Not detected | | | | | |

Stability data for the (FDC-Bilayer Variant ) bilayer tablet is given in Table 57. Conclusion: All physicochemical parameters were found to be satisfactory and comparable with the initial at 2 months under accelerated conditions. Dissolution in QC media was found to be comparable (> 90% release in 30 min). Less than 0.1% unspecified impurity of bempedoic acid was generated at 2 months under accelerated condition. Impurity profiling was found to be within specification at 2 months under accelerated conditions in accordance with ICH Q3B (R2) based on the maximum daily dose.

**Table 57: Stability Data of FDC-Bilayer Tablet (Batch no: 4759-S1-111)**

| **Sr. No.** | **Test** | | **Initial** | **Condition: 40° C / 75% RH** | |
|---|---|---|---|---|---|
| | | | | **1M** | **2M** |
| 01 | Physical appearance | | White colored, oval shaped film coated tablet debossed "000" on one side and "ABC" on other side | Complies | |
| 02 | Disintegration time (min) | | 4-5 minutes | 5-6 minutes | 2-5 minutes |
| 03 | Hardness (N) | | 119-122 | 101-150 | 126-141N |
| 04 | Water by KF (%w/v) | | 2.71 | 2.61 | 2.51 |
| 05 | Assay (%) | Ezetimibe | 99.1 | 101.8 | 103.2 |
| | | Bempedoic acid | 102.5 | 97.5 | 97.1 |

| | Impurity | Ezetimibe | | | |
|---|---|---|---|---|---|
| 06 | | Cyclic Ether | ND | BLOQ | BLOQ |
| | | EZT 21(Keto impurity) | BLOQ | BLOQ | 0.09 |
| | | Single max unspecified impurities | ND | BLOQ | BLOQ |
| | | Total impurities | BLOQ | BLOQ | 0.09 |

| | | Bempedoic acid | | | |
|---|---|---|---|---|---|
| | | Unspecified impurity | ND | ND | 0.05 |
| 07 | Dissolution | Time (min) | | | |
| For Ezetimibe: 0.45% SLS in 0.05 M acetate buffer, pH 4.5, USP Apparatus -II, 50 rpm, 500 ml | | 10 | 96.1 | 91.8 | 93.7 |
| | | 20 | 101.0 | 99.1 | 98.6 |
| | | 30 | 101.7 | 100.4 | 99.4 |
| | | 45 | 102.1 | 101.3 | 99.9 |

| | | Time (min) | | | |
|---|---|---|---|---|---|
| For bempedoic acid: 50mM phosphate buffer, pH 6.8, USP Apparatus - II, 50 rpm, 900 ml | | 10 | 72.5 | 63.3 | 71.5 |
| | | 20 | 93.2 | 88.4 | 88.2 |
| | | 30 | 99.1 | 97.5 | 94.0 |
| | | 45 | 102.4 | 103.0 | 96.5 |

| | | | | | |
|---|---|---|---|---|---|
| BLOQ- Below limit of quantification <0.05%, ND- Not detected | | | | | |

**LIST OF ABBREVIATIONS:**

| | | | |
|---|---|---|---|
| ACL | ATP citrate lyase | MCC | Microcrystalline cellulose |
| BCS | Biopharmaceutics Classification System | mg | Milligram |
| BLOQ | Below level of quantification | Min | Minute |
| cfu | Colony forming unit | mL | Milliliter |
| CQA | Critical quality attribute | MLT | Microbial Limit Test |
| CR | Child Resistant | mm | Millimeter |
| CU | Content uniformity | mM | Millimolar |
| DMF | Drug master file | mPa.s | Millipascal.second |
| DMSO | Dimethyl sulfoxide | N | Newton |
| EZT | Ezetimibe | ND | Not detected |
| FBP | Fluid Bed Processer | NDA | New drug application |
| FDC | Fixed dose combination | NF | National formulary |
| g /mol | gram per mole | NMT | Not more than |
| GMP | Good manufacturing practice | PDR | Pharmaceutical Development Report |
| HDPE | High density polyethylene | PEG | Polyethylene glycol |
| HPC-L | Hydroxypropyl cellulose low substituted | PI | Purity Index |
| HPLC | High performance liquid chromatography | PK | Pharmacokinetic |
| HPMC | Hydroxypropyl methyl cellulose | PSD | Particle size distribution |
| hrs | Hours | PVA | Polyvinyl alcohol |
| ICH | International Conference on Harmonization | QbD | Quality by design |
| IH | In house | QL | Quantification limit |
| IID: | Inactive ingredient database | QTPP | Quality target product profile |
| IR | Immediate release | RH | Relative humidity |
| LDL-C | Low-density lipoprotein cholesterol | RLD | Reference listed drug |
| RMG | Rapid mixer granulator | TAMC | Total Aerobic Microbial Count |
| RPM | Rotation per minute | TYMC | Total Yeast and Mold Count |
| SLS | Sodium lauryl sulfate | USP | United States Pharmacopeia |
| SSG | Sodium starch glycolate | XRD | X-ray diffraction |

### Example 5: ETC 1002 STUDY FOR RESOLVING PROBLEM OF STICKING; Laboratory trials

**Table 58: Formula:**

| **Ingredients** | **4490-S1-024** | **4759-S1-058** |
|---|---|---|
| | mg/unit | mg/unit |
| ETC 1002 (Bempedoic acid) | 180 | 180 |
| Colloidal silicon dioxide | - | 3 |
| Lactose monohydrate | 30 | - |
| Microcrystalline cellulose | 56 | 31 |
| Hydroxypropyl cellulose | 12 | 12 |
| Sodium Starch Glycolate | 21 | 21 |
| Ezetimibe Granules | 70 | 70 |
| colloidal silicon dioxide | 1.5 | 1 |
| Magnesium stearate | 4.5 | 4 |
| Total weight (mg) | 375 | 342.0 |

| | | |
|---|---|---|
| Manufacturing Process for formula I (4490-S1-024): | | |

All ingredients in dry mix were co-sifted and granulated with binder solution and the obtained granules were dried in a fluid bed dryer. The dried granules were blended with Ezetimibe granules along with microcrystalline cellulose, sodium starch glycolate and lubricated. The granules were then compressed into tablets.

### Manufacturing Process for formula II (4759-S1-058):

ETC 1002 and colloidal silicon dioxide were co-sifted and blended. This blend was then further mixed with microcrystalline cellulose and granulated binder solution. The granules were dried and sifted. The dried granules were blended with Ezetimibe granules along with microcrystalline cellulose, sodium starch glycolate and lubricated. The granules were then compressed into tablets.

**Table 59: OBSERVATIONS:**

| **Sticking on** | **Observation** | |
|---|---|---|
| | **formula I** | **formula II** |
| **Punch** | Sticking was observed on punch surface | Comparatively less sticking was observed |
| **Turret sticking** | Sticking was also seen on turret | |
| **Logo** | On ABC and 000 embossing sticking was observed on curve surface of letters | |

**CONCLUSION:** The surface treatment of ETC 1002 with colloidal silicon dioxide reduced sticking which was observed during compression with untreated API.

## Claims

1. A process of granulating Bempedoic acid, the process comprising:
dry mixing: Bempedoic acid, a lubricant and a pharmaceutically acceptable excipient, wherein the lubricant is colloidal silicon dioxide;
separately mixing an aqueous preparation of HPC-L;
blending the dry mixture and aqueous preparation; and
granulating the blend.

2. The process of claim 1, wherein the process further comprises using a rapid mixer to granulate the blend.

3. The process of claim 1 or 2, wherein the process further comprises drying the blend.

4. The process of any one of claims 1 to 3, wherein the process further comprises milling and co-sifting the blend.

5. The process of any one of claims 1 to 4, wherein the dry mixing is carried out for at least forty-five (45) minutes.

6. The process of claim 5, wherein the dry mixing is carried out for no more than twenty four (24) hours in duration.

7. A process of manufacturing a monolayer tablet comprising Ezetimibe and Bempedoic acid, the process comprising:
(a) a process of granulating Bempedoic acid to obtain Bempedoic acid granules, the process of granulating Bempedoic acid comprising:
dry mixing: Bempedoic acid, a lubricant and a pharmaceutically acceptable excipient, wherein the lubricant is colloidal silicon dioxide;
separately mixing an aqueous preparation of HPC-L;
blending the dry mixture and aqueous preparation; and
granulating the blend to obtain the Bempedoic acid granules;
(b) granulating a composition comprising Ezetimibe to obtain Ezetimibe granules;
(c) blending the Ezetimibe and Bempedoic acid granules together to obtain a granulated mixture;
(d) compressing the granulated mixture into a single layer; and
(e) coating the single layer to obtain the monolayer tablet.

8. The process of claim 7, wherein the process further comprises drying the tablets.

9. The process of claim 7 or 8, wherein the coating comprises one or more of: PVA, glycerol monocaprylocaprate type 1, sodium lauryl sulfate, titanium dioxide and talc.

10. The process of any one of claims 7 to 9, wherein the coating comprises each one of: PVA, glycerol monocaprylocaprate type 1, sodium lauryl sulfate, titanium dioxide and talc.

11. A process of manufacturing a bilayer tablet comprising Ezetimibe and Bempedoic acid, the process comprising:
(a) a process of granulating Bempedoic acid to obtain Bempedoic acid granules, the process of granulating Bempedoic acid comprising:
dry mixing: Bempedoic acid, a lubricant and a pharmaceutically acceptable excipient, wherein the lubricant is colloidal silicon dioxide;
separately mixing an aqueous preparation of HPC-L;
blending the dry mixture and aqueous preparation; and
granulating the blend to obtain the Bempedoic acid granules;
(b) granulating a composition comprising Ezetimibe to obtain Ezetimibe granules;
(c) blending the Ezetimibe granules with a pharmaceutically acceptable excipient;
(d) blending the Bempedoic acid granules with a pharmaceutically acceptable excipient;
(e) compressing the Ezetimibe and Bempedoic acid blends into a single composition containing two (2) separate layers; and
(f) coating the composition.

12. The process of claim 11, wherein the process further comprises drying the tablets.

13. The process of claim 11 or 12, wherein the Ezetimibe composition includes an anionic surfactant.

14. The process of any one of claims 7 to 13, wherein the process of granulating Bempedoic acid further comprises:
(a) using a rapid mixer to granulate the blend;
(b) drying the blend; and/or
(c) milling and co-sifting the blend.

15. The process of any one of claims 7 to 14, wherein the dry mixing Bempedoic acid, a lubricant and a pharmaceutically acceptable excipient is carried out for at least forty-five (45) minutes;
optionally wherein the dry mixing Bempedoic acid, a lubricant and a pharmaceutically acceptable excipient is carried out for no more than twenty four (24) hours in duration.

## Patentansprüche

1. Verfahren zum Granulieren von Bempedoinsäure, wobei das Verfahren Folgendes umfasst:
Trockenvermischen von Bempedoinsäure, einem Gleitmittel und einem pharmazeutisch annehmbaren Hilfsstoff, wobei das Gleitmittel kolloidales Siliciumdioxid ist;
getrenntes Vermischen einer wässrigen Zubereitung von HPC-L;
Mischen des Trockengemischs und der wässrigen Zubereitung; und
Granulieren der Mischung.

2. Verfahren nach Anspruch 1, wobei das Verfahren weiters die Verwendung eines Schnellmischers umfasst, um die Mischung zu granulieren.

3. Verfahren nach Anspruch 1 oder 2, wobei das Verfahren weiters das Trocknen der Mischung umfasst.

4. Verfahren nach einem der Ansprüche 1 bis 3, wobei das Verfahren weiters das Mahlen und gemeinsame Sieben der Mischung umfasst.

5. Verfahren nach einem der Ansprüche 1 bis 4, wobei das Trockenmischen zumindest fünfundvierzig (45) min lang durchgeführt wird.

6. Verfahren nach Anspruch 5, wobei das Trockenmischen nicht länger als vierundzwanzig (24) h lang durchgeführt wird.

7. Verfahren zur Herstellung einer Einschichttablette, umfassend Ezetimib und Bempedoinsäure, wobei das Verfahren Folgendes umfasst:
(a) ein Verfahren zum Granulieren von Bempedoinsäure, um ein Bempedoinsäure-Granulat zu erhalten, wobei das Verfahren zum Granulieren von Bempedoinsäure Folgendes umfasst:
Trockenmischen von Bempedoinsäure, einem Gleitmittel und einem pharmazeutisch annehmbaren Hilfsstoff, wobei das Gleitmittel kolloidales Siliciumdioxid ist;
getrenntes Vermischen einer wässrigen Zubereitung von HPC-L;
Mischen des Trockengemischs und der wässrigen Zubereitung; und
Granulieren der Mischung, um das Bempedoinsäure-Granulat zu erhalten;
(b) Granulieren einer Zusammensetzung, umfassend Ezetimib, um ein Ezetimib-Granulat zu erhalten;
(c) Zusammenmischen des Ezetimib- und des Bempedoinsäure-Granulats, um ein granuliertes Gemisch zu erhalten;
(d) Komprimieren des granulierten Gemischs zu einer einzelnen Schicht; und
(e) Aufschichten der einzelnen Schicht, um die Einschichttablette zu erhalten.

8. Verfahren nach Anspruch 7, wobei das Verfahren weiters das Trocknen der Tabletten umfasst.

9. Verfahren nach Anspruch 7 oder 8, wobei die Beschichtung eines oder mehrere der Folgenden umfasst: PVA, Glycerinmonocaprylocaprat Typ 1, Natriumlaurylsulfat, Titandioxid und Talk.

10. Verfahren nach einem der Ansprüche 7 bis 9, wobei die Beschichtung jedes der Folgenden umfasst: PVA, Glycerinmonocaprylocaprat Typ 1, Natriumlaurylsulfat, Titandioxid und Talk.

11. Verfahren zur Herstellung einer Zweischichttablette, umfassend Ezetimib und Bempedoinsäure, wobei das Verfahren Folgendes umfasst:
(a) ein Verfahren zum Granulieren von Bempedoinsäure, um ein Bempedoinsäure-Granulat zu erhalten, wobei das Verfahren zum Granulieren von Bempedoinsäure Folgendes umfasst:
Trockenmischen von Bempedoinsäure, einem Gleitmittel und einem pharmazeutisch annehmbaren Hilfsstoff, wobei das Gleitmittel kolloidales Siliciumdioxid ist;
getrenntes Vermischen einer wässrigen Zubereitung von HPC-L;
Mischen des Trockengemischs und der wässrigen Zubereitung; und
Granulieren der Mischung, um das Bempedoinsäure-Granulat zu erhalten;
(b) Granulieren einer Zusammensetzung, umfassend Ezetimib, um ein Ezetimib-Granulat zu erhalten;
(c) Mischen des Ezetimib-Granulats mit einem pharmazeutisch annehmbaren Hilfsstoff;
(d) Mischen des Bempedoinsäure-Granulats mit einem pharmazeutisch annehmbaren Hilfsstoff;
(e) Komprimieren der Ezetimib- und Bempedoinsäure-Mischungen zu einer einzelnen Zusammensetzung, die zwei (2) getrennte Schichten enthält; und
(f) Aufschichten der Zusammensetzung.

12. Verfahren nach Anspruch 11, wobei das Verfahren weiters das Trocknen der Tabletten umfasst.

13. Verfahren nach Anspruch 11 oder 12, wobei die Ezetimib-Zusammensetzung ein anionisches Tensid umfasst.

14. Verfahren nach einem der Ansprüche 7 bis 13, wobei das Verfahren zum Granulieren von Bempedoinsäure weiters Folgendes umfasst:
(a) Verwenden eines Schnellmischers, um die Mischung zu granulieren;
(b) Trocknen der Mischung; und/oder
(c) Mahlen und gemeinsames Sieben der Mischung.

15. Verfahren nach einem der Ansprüche 7 bis 14, wobei das Trockenmischen von Bempedoinsäure, einem Gleitmittel und einem pharmazeutisch annehmbaren Hilfsstoff zumindest fünfundvierzig (45) min lang durchgeführt wird;
wobei das Trockenmischen von Bempedoinsäure, einem Gleitmittel und einem pharmazeutisch annehmbaren Hilfsstoff nicht länger als vierundzwanzig (24) h lang durchgeführt wird.

## Revendications

1. Procédé de granulation d'acide bempédoïque, le procédé comprenant les étapes consistant à :
mélanger à sec : de l'acide bempédoïque, un lubrifiant et un excipient pharmaceutiquement acceptable, dans lequel le lubrifiant est le dioxyde de silicium colloïdal ;
mélanger séparément une préparation aqueuse de HPC-L ;
mélanger le mélange sec et la préparation aqueuse ; et
granuler le mélange.

2. Procédé selon la revendication 1, dans lequel le procédé comprend en outre l'utilisation d'un mélangeur rapide pour granuler le mélange.

3. Procédé selon la revendication 1 ou 2, dans lequel le procédé comprend en outre un séchage du mélange.

4. Procédé selon l'une quelconque des revendications 1 à 3, dans lequel le procédé comprend en outre le broyage et le co-tamisage du mélange.

5. Procédé selon l'une quelconque des revendications 1 à 4, dans lequel le mélange à sec est effectué pendant au moins quarante-cinq (45) minutes.

6. Procédé selon la revendication 5, dans lequel le mélange à sec est effectué pendant une durée n'excédant pas vingt-quatre (24) heures.

7. Procédé de fabrication d'un comprimé monocouche comprenant de l'ézétimibe et de l'acide bempédoïque, le procédé comprenant :
(a) un procédé de granulation d'acide bempédoïque afin d'obtenir des granulés d'acide bempédoïque, le procédé de granulation d'acide bempédoïque comprenant les étapes consistant à :
mélanger à sec : de l'acide bempédoïque, un lubrifiant et un excipient pharmaceutiquement acceptable, dans lequel le lubrifiant est le dioxyde de silicium colloïdal ;
mélanger séparément une préparation aqueuse de HPC-L ;
mélanger le mélange sec et la préparation aqueuse ; et
granuler le mélange afin d'obtenir les granulés d'acide bempédoïque ;
(b) granuler une composition comprenant de l'ézétimibe afin d'obtenir des granulés d'ézétimibe ;
(c) mélanger les granulés d'ézétimibe et d'acide bempédoïque afin d'obtenir un mélange granulé ;
(d) comprimer le mélange granulé en une couche unique ; et
(e) appliquer en revêtement la couche unique afin d'obtenir le comprimé monocouche.

8. Procédé selon la revendication 7, dans lequel le procédé comprend en outre le séchage des comprimés.

9. Procédé selon la revendication 7 ou 8, dans lequel le revêtement comprend un ou plusieurs parmi : de l'APV, du monocaprylocaprate de glycérol de type 1, du laurylsulfate de sodium, du dioxyde de titane et du talc.

10. Procédé selon l'une quelconque des revendications 7 à 9, dans lequel le revêtement comprend chacun parmi : de l'APV, du monocaprylocaprate de glycérol de type 1, du laurylsulfate de sodium, du dioxyde de titane et du talc.

11. Procédé de fabrication d'un comprimé bicouche comprenant de l'ézétimibe et de l'acide bempédoïque, le procédé comprenant :
(a) un procédé de granulation d'acide bempédoïque afin d'obtenir des granulés d'acide bempédoïque, le procédé de granulation d'acide bempédoïque comprenant les étapes consistant à :
mélanger à sec : de l'acide bempédoïque, un lubrifiant et un excipient pharmaceutiquement acceptable, dans lequel le lubrifiant est le dioxyde de silicium colloïdal ;
mélanger séparément une préparation aqueuse de HPC-L ;
mélanger le mélange sec et la préparation aqueuse ; et
granuler le mélange afin d'obtenir les granulés d'acide bempédoïque ;
(b) granuler une composition comprenant de l'ézétimibe afin d'obtenir des granulés d'ézétimibe ;
(c) mélanger les granulés d'ézétimibe avec un excipient pharmaceutiquement acceptable ;
(d) mélanger les granulés d'acide bempédoïque avec un excipient pharmaceutiquement acceptable ;
(e) comprimer les mélanges d'ézétimibe et d'acide bempédoïque en une seule composition contenant deux (2) couches distinctes ; et
(f) appliquer la composition en revêtement.

12. Procédé selon la revendication 11, dans lequel le procédé comprend en outre le séchage des comprimés.

13. Procédé selon la revendication 11 ou 12, dans lequel la composition d'ézétimibe inclut un tensioactif anionique.

14. Procédé selon l'une quelconque des revendications 7 à 13, dans lequel le procédé de granulation d'acide bempédoïque comprend en outre les étapes consistant à :
(a) utiliser un mélangeur rapide pour granuler le mélange ;
(b) sécher le mélange ; et/ou
(c) broyer et co-tamiser le mélange.

15. Procédé selon l'une quelconque des revendications 7 à 14, dans lequel le mélange à sec d'acide bempédoïque, d'un lubrifiant et d'un excipient pharmaceutiquement acceptable est effectué pendant au moins quarante-cinq (45) minutes ;
facultativement dans lequel le mélange à sec d'acide bempédoïque, d'un lubrifiant et d'un excipient pharmaceutiquement acceptable est effectué pendant une durée maximale de vingt-quatre (24) heures.
